(19) Europäisches Patentamt
European Patent Office
Office européen des brevets

(11) **EP 4 729 534 A1**

(12) **EUROPEAN PATENT APPLICATION**

(43) Date of publication:
**22.04.2026 Bulletin 2026/17**

(21) Application number: **25209828.0**

(22) Date of filing: **20.10.2025**

(51) International Patent Classification (IPC):
***C07K 16/00*** (2006.01)   ***C07K 17/00*** (2006.01)

(52) Cooperative Patent Classification (CPC):
**C07K 16/00; C07K 17/00;** C07K 2317/92;
C07K 2319/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **21.10.2024 EP 24207783**

(71) Applicant: **Technische Universität München, in
Vertretung des Freistaats Bayern
80333 München (DE)**

(72) Inventors:
• **BERENSMEIER, Sonja
81829 Munich (DE)**
• **BAGHBADERANI, Yasmin Kaveh
72020 Tübingen (DE)**
• **SCHWAMINGER, Sebastian
8010 Graz (AT)**

(74) Representative: **Schiweck Weinzierl Koch
Patentanwälte Partnerschaft mbB
Ganghoferstraße 68 B
80339 München (DE)**

Remarks:
The complete document including Reference
Table(s) and the Sequence Listing(s) can be
downloaded from the EPO website

(54) **POLYPEPTIDES COMPRISING PROTEIN DOMAINS LINKED VIA RIGID AMINO ACID LINKERS**

(57) The present invention relates to a novel polypeptide that contains protein domains capable of binding to a specific site of the surface of a target protein, wherein each protein domain is translationally fused in its N- to C-terminal orientation to a further protein domain that is also in its N- to C-terminal orientation, via a rigid amino acid linker keeping a fixed distance between said protein domains and said rigid amino acid linker is also in its N- to C-terminal orientation. The present invention further relates to a method for producing that polypeptide, comprising coupling that polypeptide to a carrier material, wherein said carrier material is a particle, bead, resin, membrane, solid support or tag. The present invention further relates to a method for producing a purified target protein comprising eluting a target protein bound to the polypeptide of the present invention.

**Description**

The present application claims the benefit of priority of EP Patent Application No. 24207783.2 filed 21 October 2024, the content of which is hereby incorporated by reference in its entirety for all purposes.

The present invention relates to a polypeptide, wherein said polypeptide comprises protein domains binding to a specific site on the surface of a target protein, wherein each protein domain is translationally fused in its N- to C-terminal orientation to a further protein domain that is also in its N-to C-terminal orientation, via a rigid amino acid linker keeping a fixed distance between said protein domains and said rigid amino acid linker is also in its N- to C-terminal orientation, wherein said polypeptide is coupled to a carrier material, wherein said carrier material is a particle, bead, resin, membrane, solid support or tag. The present invention further relates to a method for producing the polypeptide of the present invention, comprising coupling the polypeptide to a suitable carrier material. Furthermore, the present invention relates to a method for producing a purified target protein, comprising eluting a target protein bound to the polypeptide of the present invention.

Proteins binding domains have found widespread use in biotechnology, owing to their capacity to attach to a desired molecule with high specificity and affinity. For example, they see widespread use in purifying and/or precipitating biomolecules during production, along with other applications. Examples of binding domains highly amenable to application in biotechnology include, but are not limited to, IgG binding domains, IgA binding domains, and IgM binding domains. These binding domains refer to binding domains capable of binding to IgG/A/M antibodies and are regularly utilized in the purification of antibodies, which are becoming increasingly important as therapeutic agents (Ribatti, 2014). Proteins containing such IgG/M/A binding domains can be found in nature, specifically, within the host-evasion systems of the *Streptococcus* and *Peptostreptococcus* genera of bacteria. Examples include Proteins A and G *(Staphylococcus aureus)* and Protein L *(Peptostreptococcus magnus),* which respectively have highest binding affinity for IgG, IgA, and IgM antibody Fc constant regions. Protein A is capable of binding to the Fc region of all human antibodies, except IgG3, and certain animal antibodies. Specifically, Protein A contains 5 homologous immunoglobulin binding domains, domains A to E. Protein A chromatography, also termed "affinity chromatography", is a key step in antibody production. Affinity chromatography has proven highly efficacious in purifying antibodies, e.g. mAbs, due to the high affinity of Protein A's binding domains, resulting in purities of over 95%. However, the low binding capacity of Protein A as currently applied to affinity chromatography means that this process acts as a bottleneck in antibody production methods, as the low binding capacity means that a given fermentation batch must undergo several cycles of affinity chromatography to achieve optimal purity. Extensive work has gone into overcoming this hurdle by optimizing immunoglobulin binding domains, e.g. Protein A, by, e.g. the introduction of point mutations to alter its binding capacity or stability or by polymerization of Protein A to produce fusion proteins typically consisting of 4-6 Protein A domains joined by peptide linkers (Kanje et al., 2020; Pabst et al., 2018). Similar issues have arisen with other binding domains used for the purification of a protein of interest. Whilst these approaches have provided some improvements, further work is needed to provide further gains in binding capacity of protein domains.

Accordingly, there is still a need to provide further means and methods for the purification of biomolecules, such as proteins. The present invention therefore addresses this need and provides a solution as described herein, defined in the claims, demonstrated in the examples and illustrated in the figures herein

Namely, the present invention provides a polypeptide, wherein said polypeptide comprises protein domains binding to a specific site on the surface of a target protein, wherein in N- to C-terminal direction said protein domains are translationally fused via a rigid amino acid linker keeping a fixed distance between said protein domains, wherein said polypeptide is coupled to a carrier material.

Specifically, in the polypeptide of the present invention each protein domain is translationally fused to a further protein domain via said rigid amino acid linker.

The term "polypeptide" when used herein refers to a molecule comprising a polymer of amino acids linked together by a peptide bond(s). Said term is herein interchangeably used with the term "protein". Polypeptides include polypeptides and peptides of any length. A "polypeptide" as used herein encompasses both naturally-occurring and non-naturally-occurring amino acids. In the polypeptide notation used herein, the left-hand direction is the amino terminal direction and the righthand direction is the carboxy-terminal direction, in accordance with standard usage and convention. The terms "polypeptide" and "protein" also refer to naturally or non-naturally modified polypeptides/proteins wherein the modification is affected e.g. by glycosylation, acetylation, phosphorylation and the like. Such modifications are well known in the art. Further, as is described herein, a polypeptide of the present invention may comprise a number of protein domains. Encompassed by the term "polypeptide" or "protein" is the polypeptide of the present invention as well as a protein domain comprised by the polypeptide of the present invention.

A "protein domain binding to a specific site on the surface of a target protein" (or the plural thereof) is also referred to herein as "protein binding domain", "protein domain" or "domain". Said terms may be interchangeably used in the context of the present application. The term "protein domain" generally refers to any naturally-occurring or non-naturally occurring molecule or portion thereof capable of binding a protein or peptide, e.g. a target protein as referred to herein,

using specific intermolecular interactions.

A protein domain is a part of the polypeptide of the present invention. It forms a tertiary structure which is independent of the rest of the polypeptide of the present invention. Upon binding, a protein domain may undergo a conformational change. A protein domain usually forms a functional unit. Because a protein domain is independently stable, protein domains can be "swapped" by genetic engineering between one protein and another. In fact, the present invention makes use of this principle and swaps protein domains from its natural context into a heterologous context, e.g. by swapping them into the polypeptide of the present invention. However, protein domains used in the context of the present invention can also be generated de novo, e.g. peptide aptamers as described herein and known in the art or as described in Cao et al., 2022. Preferred protein domains are described hereinbelow.

The term "conformation" or "conformational state" of proteins (or a protein domain) generally refers to the range of structures that a protein (or a protein domain) can assume at any given moment. Those skilled in the art will recognize that conformation or conformational state determinants include the primary structure of the protein (or a protein domain) and the environment surrounding the protein (or a protein domain) as reflected in the protein's (or a protein domain's) amino acid sequence (including modified amino acids). Conformation or conformational of a protein (or a protein domain) include secondary structure (e.g., $\alpha$-helix, $\beta$-sheet, among others), tertiary structure (e.g., three-dimensional folding of a polypeptide chain) and quaternary structure (e.g., a polypeptide chain) and interactions with other protein subunits. Post-translational and other modifications to the polypeptide chain such as ligand binding- here binding of a target protein, phosphorylation, sulfation, glycosylation or attachment of hydrophobic groups, among others, can affect conformation. Moreover, environmental factors such as pH, salt concentration, ionic strength and osmotic pressure of the surrounding solution, among others, and interactions with other proteins and cofactors can affect conformation.

By" a specific site on the surface of a target protein " herein is meant a region within the amino acid sequence of a target protein that interacts with a protein binding domain of the polypeptide of the invention. As described herein, a protein binding domain is capable of binding a protein or peptide, e.g. a target protein as referred to herein using specific intermolecular interactions. Such a region within the amino acid sequence of a target protein may be an antigenic determinant of a target protein. Such a region can include three amino acids in a spatial conformation unique to the region. Generally, such a region may comprise at least 4, 5, 6, 7 or more amino acids, more usually at least 8, 9, or 10 such amino acids. Such a region may be linear, i.e., the amino acids may be consecutive (or contiguous) or it may be conformational. "Conformational " when used in the context of such a region means a region that includes amino acids in a spatial conformation that is unique to the folded, three-dimensional conformation of the target protein. In general, a conformational region comprises amino acids that are non-contiguous in linear sequence that come together in the folded structure of the target protein. However, a conformational region may also comprise a linear sequence of amino acids (not present in the denatured state) in a conformational form unique to the three-dimensional folded conformation of the target protein.

The binding of a protein domain to a specific site on the surface of a target protein is ideally specific. The terms "specifically binding" and "specific binding" as used herein refer to the ability of a protein domain to preferentially bind to a target protein. Preferably, specific binding discriminates between desirable and undesirable target proteins. The terms "specifically bind", "selectively bind", "preferentially bind" and their grammatical equivalents are used interchangeably herein.

The protein domains comprised by the polypeptide of the present invention are translationally fused via a rigid amino acid linker. This means that each protein domain is fused to a further protein domain via a rigid amino acid linker. Put differently, the rigid amino acid linker is, so to say, between two (and each further) protein domain(s). Accordingly, each protein domain is fused to a further via a rigid amino acid linker. "Translational fusion", as used herein and in the context of the present invention, refers to joining two or more protein domains by producing a genetic construct that encodes both the two or more protein domains and the intermediately placed rigid amino acid linker, so as to produce a single continuous polypeptide chain. Such a genetic construct, so to say, fuses the protein domains and the rigid amino acid linker in frame. The term "in frame" means that two or more nucleotide sequences are covalently linked together by 5'-3' bonds of the sugar backbone of a nucleic acid such that these two or more nucleotide sequences are in the same open reading frame which is transcribed and then translated as one entity. This allows for the polypeptide of the present invention to be expressed without the need for additional processing steps post-expression and greatly simplifies both the construction of a genetic construct encoding the fusion protein and the protein folding process that follows expression.

By "N- to C-terminal direction" in the context of the protein domains comprised by the polypeptide of the present invention is meant that each protein domain is fused in its N- to C-terminal orientation to a further protein domain that is also in its N- to C-terminal orientation, via a rigid amino acid linker which is also in its N- to C-terminal orientation. Indeed, by convention, amino acid sequences are written N-terminus to C-terminus, left to right. This correlates the translation direction to the text direction, because when a protein is translated from messenger RNA, it is created from the N-terminus to the C-terminus, as amino acids are added to the carboxyl end of the protein.

A "target protein" in the context of the present invention may be any protein which is capable of binding to a protein domain comprised by the polypeptide of the present invention. A target protein may be glycosylated. The target protein may be displayed by a virus, a bacterial cell, fungal cell or a mammalian cell. Of course, the target protein does not have to be displayed as described before, the target protein may also be "free", e.g. may be in a solution. An example of a target protein may be an immunoglobulin, e.g. an antibody, Fab fragment or Fc-fusion protein. For example, if the target protein is displayed by a virus, e.g. an AAV, a PKD domain may be used as protein domain comprised by the polypeptide of the present invention. For example, if the target protein is glycosylated, a lectin may be used as a protein domain comprised by the polypeptide of the present invention. For example, if the target protein is an immunoglobulin, an IgG binding domain, IgA binding domain, or IgM binding domain may be used as protein domain comprised by the polypeptide of the present invention.

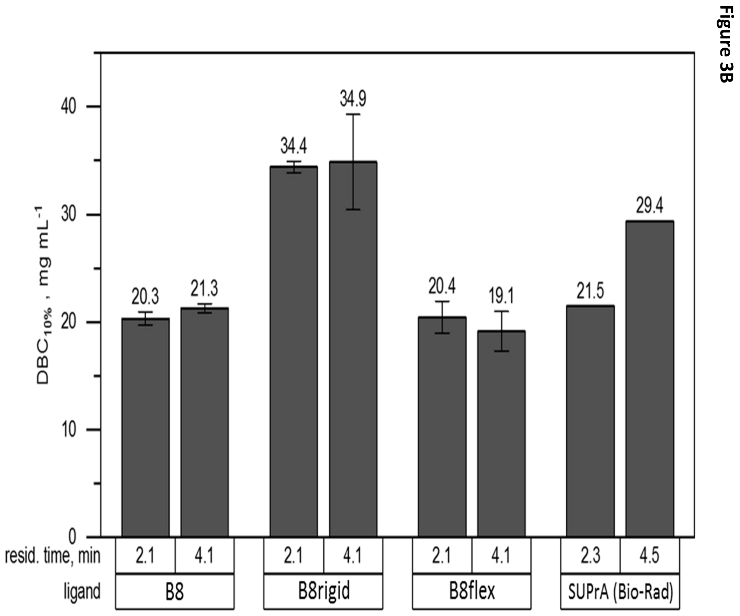

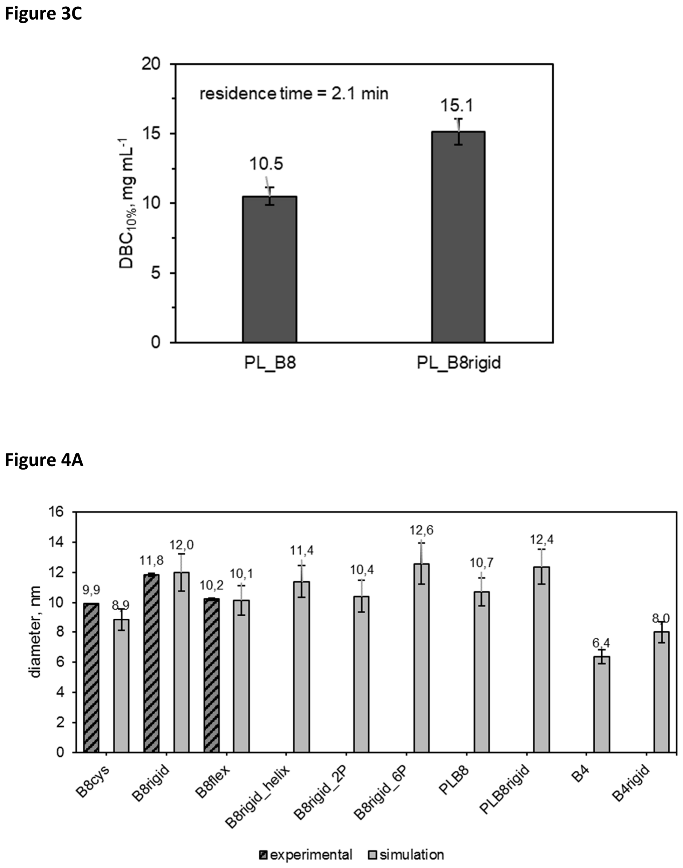

By providing the polypeptide of the present invention comprising protein binding domains translationally fused with a rigid amino acid linker, the present inventors have demonstrated a novel solution to improve, e.g. dynamic binding capacity, thereby, e.g. increasing the speed and efficiency of biotechnology protocols that require the use of protein binding domains. This is demonstrated in **Figure 3B.** In fact, use of a rigid amino acid linker ("B8rigid") which fuses B-domains of protein A (as an example of a protein binding domain as used herein) provides for an improved dynamic binding capacity in contrast to the use of flexible amino acid linkers ("B8", "B8flex"). It is also apparent from **Figure 3B** that the use of a rigid linker provides for an improved dynamic binding capacity when compared to a conventional protein A chromatography column ("SUPrA"). Furthermore, Figure 3C shows also an increase in binding capacity for polymerised domains from Protein L (PL_B8rigid) compared to the wildtype linker with no addition (PL_B8).

The term "dynamic binding capacity" as used herein, refers to the amount of protein of interest that can bind to a polypeptide of the present invention, e.g. coupled to a carrier, such as a chromatography matrix under a constant flow without having a significant amount of protein of interest in the flow through. The dynamic binding capacity can be determined by loading a sample containing a known concentration of protein of interest. The load of the protein sample on the column is monitored and will bind to the matrix to a certain break point before unbound protein will flow through the matrix. From the breakthrough curve at a loss of, for example 10% protein, the dynamic binding capacity is found and the experiment is stopped. The dynamic binding capacity may be defined by the amount of protein of interest that can bind to the polypeptide of the present invention, e.g. coupled to a carrier under a constant flow with not more than 5%, 6%, 7%, 8%, 9%, 10%, 12%, 15%, 17% or 20% of the protein of interest lost in the flow-through, preferably of the concentration of protein of interest being loaded at the same point in time.

The polypeptide of the present invention may be used for the purpose of protein chromatography, e.g. to enrich, isolate or purify a target protein, e.g. an immunoglobulin, such as an antibody. The term "protein chromatography," as used herein, refers to a dynamic separation technique which separates or enriches i.e. purifies a target protein referred to herein (e.g. an immunoglobulin) from other molecules in the mixture and allows it to be isolated. Typically, in a protein chromatography method, a mobile phase (e.g. liquid) transports a sample containing the analyte of interest across or through a stationary phase (normally solid) medium. The polypeptide of the present invention is coupled to a carrier material. The carrier material may be part of the stationary phase and, thus, the polypeptide of the present invention may be part of the stationary phase. Differences in partition or affinity to the stationary phase, also including the carrier material to which the polypeptide of the present invention is coupled to, separate different analytes, while the mobile phase carries the different analytes out at different time. An "analyte" or "analyte of interest" when used herein includes a target protein as described herein which is bound by a polypeptide of the present invention.

A preferred mode of protein chromatography in the context of the present invention is affinity chromatography. Accordingly, the polypeptide of the present invention may be applied for the purpose of affinity chromatography. The term "affinity chromatography," as used herein, refers to a mode of chromatography where the target protein to be separated or enriched, e.g. purified, is isolated by its interaction with the polypeptide of the present invention which binds to a specific site on the surface of a target protein. Accordingly, a protein domain comprised by the polypeptide of the present invention specifically interacts with a target protein as described herein. In one embodiment, affinity chromatography involves the addition of a sample containing a target protein (e.g. an immunoglobulin) to a solid support which carries on it the polypeptide of the present invention as described herein. A preferred mode of affinity chromatography in the context of the present invention is protein A affinity chromatography, protein G affinity chromatography, or protein L affinity chromatography. Accordingly, the polypeptide of the present invention may be applied for the purpose of protein A affinity chromatography, protein G affinity chromatography, or protein L affinity chromatography.

The term "protein A affinity chromatography," as used herein, refers to the separation or isolation of a target protein using protein A, e.g. the entire protein A or domains thereof which bind immunoglobulins, such as those described herein. Protein A or a domain thereof bind immunoglobulins and, thus, allow, e.g. the purification of immunoglobulins. Accordingly, a preferred protein domain comprised by the polypeptide of the present invention is a domain from protein A as described herein. Examples of protein A affinity chromatography media/resin known in the art include those having the protein A immobilized onto a controlled pore glass backbone, e.g. PROSEP A™ and PROSE vA™ media/resin (Millipore); those having protein A immobilized onto a polystyrene solid phase, e.g. the POROS 50A™ and Poros MabCapture A™ media/resin (Applied Biosystems, Inc.); and those having protein A immobilized on an agarose solid support, e.g. PROTEIN A SEPHAROSE FAST FLOW™ or MABSELECT™ columns (Amersham Biosciences). Each of these media/resin may be equipped with the polypeptide of the present invention which provides for an improvement over conventional protein A media/resin as is shown in Figure 3B (cf. "B8-rigid" versus "SUPrA").

The term "immunoglobulin," "Ig" or "antibody" (used interchangeably herein) refers to a protein having a basic four-polypeptide chain structure consisting of two heavy and two light chains, said chains being stabilized, for example, by interchain disulfide bonds, which has the ability to specifically bind an antigen. Immunoglobulins or antibodies may be monoclonal or polyclonal and may exist in monomeric or polymeric form, for example, IgM antibodies which exist in pentameric form and/or IgA antibodies which exist in monomeric, dimeric or multimeric form. The term "fragment" refers to a part or portion of an antibody or antibody chain comprising fewer amino acid residues than an intact or complete antibody or antibody chain. Fragments can be obtained via chemical or enzymatic treatment of an intact or complete antibody or antibody chain. Fragments can also be obtained by recombinant means. Fragments can include, but are not limited to, antigen-binding fragments (Fab), divalent antigen-binding fragments (F(ab)2), single chain variable fragments (scFv) nanobodies, diabodies, and bispecific T-cell engagers. All of these fragments retain some aspect of antibody function, whilst lacking their complete structure. Their size and versatility mean they have widespread uses in both biotechnology and medicine. The term "monoclonal antibody" refers to an antibody originating from a single clone of immune cells.

An "Fc-fusion protein" as referred to herein comprises an Fc-region. The Fc-region may be a native-sequence Fc region or a "variant Fc-region". A "native-sequence Fc region" comprises an amino acid sequence identical to the amino acid sequence of an Fc region found in nature. Native-sequence human Fc regions include, for example, a native-sequence human IgGI Fc region (non-A and A allotypes); native-sequence human IgG2 Fc region; native-sequence human IgG3 Fc region; and native-sequence human IgG4 Fc region, as well as naturally occurring variants thereof. A "variant Fc region" comprises an amino acid sequence that differs from that of a native sequence Fc region by virtue of at least one amino acid modification, preferably one or more amino acid substitution(s). Preferably, the variant Fc region has at least one amino acid substitution compared to a native-sequence Fc region or to the Fc region of a parent polypeptide, e.g., from about one to about ten amino acid substitutions, and preferably from about one to about five amino acid substitutions in a native-sequence Fc region or in the Fc region of the parent polypeptide. The variant Fc region herein will preferably possess at least about 80% identity with a native-sequence Fc region and/or with an Fc region of a parent polypeptide, and most preferably at least about 90% identity therewith, more preferably at least about 95% identity therewith.

The term "protein G affinity chromatography," as used herein, refers to the separation or isolation of a target protein using protein G, e.g. the entire protein G or domains thereof which bind immunoglobulins, such as those described herein. Protein G or a domain thereof bind immunoglobulins and, thus, allow, e.g. the purification of immunoglobulins. While protein A has a higher affinity for IgG antibodies from certain species (such as human, rabbit, and pig), protein G offers broader species reactivity, making it particularly useful for purifying antibodies from a wider range of sources, including mouse, rat, and bovine. Recombinant forms of protein G have also been engineered to optimize its binding properties and enhance its effectiveness in various processes. Accordingly, a preferred protein domain comprised by the polypeptide of the present invention is a domain from protein G as described herein.

The term "protein L affinity chromatography," as used herein, refers to the separation or isolation of a target protein using protein L, e.g. the entire protein L or domains thereof which bind immunoglobulins, such as those described herein. Protein L or a domain thereof bind immunoglobulins and, thus, allow, e.g. the purification of immunoglobulins. Protein L is unique in its ability to bind to the light chain of immunoglobulins (Ig) from various species, regardless of the species' heavy-chain subclass. This characteristic sets protein L apart from other antibody-binding proteins like protein A and protein G, which primarily bind to the Fc region of IgG antibodies. Due to its ability to interact with the variable region of immunoglobulin light chains, protein L is particularly useful for the purification and detection of antibodies that might not bind well to protein A or protein G, such as camelid antibodies (e.g., llama, alpaca) that lack a traditional Fc region. Recombinant forms of protein L have also been engineered to optimize its binding properties and enhance its effectiveness in various processes. Accordingly, a preferred protein domain comprised by the polypeptide of the present invention is a domain from protein L as described herein. Applications of antibody binding proteins include antibody purification and immunoprecipitation. For such uses, the antibody binding proteins are first immobilized on a suitable substrate, with the binding regions exposed to the antibody-containing solution.

Purification of a target protein, e.g. an immunoglobulin, is the isolation of a target protein from, e.g. the cell culture medium or supernatant in which it was expressed. A polypeptide of the present invention may typically be immobilized on a carrier, e.g. chromatography resin to which it is attached, e.g. by covalent bonding, such as cross-linked agarose, silica, cellulose etc. When solutions containing the target protein are passed over the immobilized polypeptide of the present invention, they will be bound by the polypeptide, allowing for subsequent extraction and purification. Immobilization of a polypeptide of the present invention to a suitable carrier is typically achieved through (enzymatic) covalent linkage of the polypeptide to the carrier, with adsorption, entrapment, and polymerization also commonly used (Arora et al., 2017; Fishman and Berg, 2019; Matsuda, 2022). Some methods result in randomly oriented immobilization of the polypeptide of the present invention, in which immobilized polypeptides are bonded via exposed side chain amino acids to different sites along the surface of the carrier. This can limit available binding domains, but can be prevented through orientation directed immobilization that can be further enhanced through the use of linkers to maintain orientation and optimal inter-protein distance (Shen et al., 2017; Wilchek and Miron, 2003). Once the target protein containing solution has passed over the, e.g. chromatography resin and been bound by the polypeptide of the present invention, the target protein must be dissociated from said polypeptide protein. This is achieved through a number of methods commonly known in the art.

The polypeptide of the present invention may also be used in immunoprecipitation. Immunoprecipitation is a method that allows a specific target protein to be isolated and purified. Immunoprecipitation follows the same principles that target protein purification relies on. The polypeptide of the present invention may be immobilized to a carrier, e.g. beads rather than a resin, allowing for high binding surface area to be achieved upon application of the beads to the target protein containing solution. Once the polypeptide-target protein complex is bound, the solution can be washed away and dissociation of the complex in order to isolate the target protein can be achieved through, e.g. altering pH and/or osmolarity.

The term "protein A" (or "SpA") as used herein may refer to a surface protein derived from the bacterium *Staphylococcus aureus.* Said term also includes a domain of protein A, e.g. a domain of protein A as described herein. Protein A has the unique ability to bind to the constant region (Fc region) of immunoglobulins, particularly immunoglobulin G (IgG), from various species. Protein A (e.g., having Uniprot Accession Number: P02976) is commonly used in affinity chromatography to purify antibodies. A preferred Protein A in the context of the present invention is shown in SEQ ID NO: 1. The interaction between protein A and antibodies can also be exploited for antibody detection, immunoprecipitation, and/or other applications. Additionally, recombinant forms of protein A are engineered to optimize binding efficiency and specificity, enhancing its utility in various processes. The amino-terminal region features five immunoglobulin-binding domains, labelled E, D, A, B, and C (Sjödahl, 1977; Uhlén et al., 1984). The B-domain is the most commonly used binding domain when individual domains are applied in biotechnology due to its high affinity for the Fc region of human IgG antibodies. Each domain comprises approximately 58 amino acids, with the 5 domains exhibiting 65-90% amino acid sequence homology with one another. The Z domain of SpA is an engineered variant of the B domain, with an alanine replacing a glycine at position 29 (Nilsson et al., 1987). The E, D, A, B, and C domains of SpA each have specific Ig-binding sites. Reports indicate that each domain features a Fab binding site. The non-Ig binding segment of SpA is located at the C-terminus and is referred to as the X region or X-domain. Crucially, SpA domains can bind antibodies without significantly altering the antibodies' affinity for antigens. The aforementioned domains are encompassed by the term "protein A" when used herein. Preferred domains of Protein A are described herein and shown in SEQ ID NOs 2 to 6.

The term "protein G" (or "SpG") as used herein may refer to bacterial surface protein originally isolated from certain strains of bacteria, primarily of the species *Streptococcus.* Said term also includes a domain of protein G, e.g. a domain of protein G as described herein Similar to protein A, protein G possesses the ability to bind to the Fc region of immunoglobulins (IgG antibodies), making it a valuable tool for purification. Protein G (e.g., having Uniprot Accession Number: P06654) can be used in affinity chromatography to purify antibodies from complex samples. Protein G plays a similar biological function to Protein A, acting to bind immunoglobulins so as to prevent opsonization of the bacterial cell by antibodies. SpG binds to antibodies of all four human IgG subclasses via the Fab or Fc region, with considerably higher binding affinity displayed towards the Fc region. In addition to a shared functional role in bacterial host immune evasion, SpG also shares many structural similarities with SpA. SpG is anchored to the bacterial cell wall and possesses two to three immunoglobulin-binding B domains, each of 55 amino acids in length, comparable in structure to the immunoglobulin-binding domains of SpA. SpG B domains target the CH2-CH3 interface of the Fc region of IgG, with each domain capable of independently binding an IgG molecule. Despite these similarities, the binding domains of SpA and SpG are non-homologous in sequence, with the secondary structure of SpA and SpG also differing. SpA comprises three $\alpha$-helices whilst SpG comprises two $\beta$-sheets connected by an $\alpha$-helix (Housden et al., 2003). Furthermore, SpG is capable of binding to IgG from a wider range of animal species compared to SpA, making it more amenable to use in methods involving non-human antibodies. In addition to IgG binding domains, SpG also possesses a serum albumin binding domain, which is often removed from versions of SpG used in biotechnology functions to prevent unwanted albumin binding. Protein G has many applications in biotechnology and protein isolation methods, as discussed herein. Such applications of Protein G are amenable to benefiting from the present invention. The aforementioned domains of Protein G are encompassed by the term "protein G" when used herein. Accordingly, a preferred protein domain comprised by the polypeptide of the present invention is a domain from Protein G as described herein.

The term "protein L" (or "PpL") as used herein may refer to a bacterial surface protein derived from certain strains of bacteria, such as *Peptostreptococcus magnus.* Protein L is unique in its ability to bind to the light chain of immunoglobulins (Ig) from various species, regardless of the species' heavy-chain subclass. This characteristic sets protein L (e.g., having Uniprot Accession Number: Q53291) apart from other antibody-binding proteins like protein A and protein G, which primarily bind to the Fc region of IgG antibodies. Due to its ability to interact with the variable region of immunoglobulin light chains, protein L is particularly useful for the purification and detection of antibodies that might not bind well to protein A or protein G, such as camelid antibodies (e.g., llama, alpaca) that lack a traditional Fc region. As with SpA and SpG, Protein L binds to immunoglobulins via repeated domains, with Protein L possessing up to five homologous light-chain binding B domains. These B domains are non-homologous with immunoglobulin binding domains present in SpA and SpG, which is unsurprising given that they target the $\kappa$-light chain rather than the heavy chain of immunoglobulins. Protein L shares its secondary structure with SpG despite a low sequence homology, comprising two $\beta$-sheets joined by an $\alpha$-helix. The $\kappa$-light

chain binding domain is comprised of 12 residues located across one of the β-sheets and the α-helix (Housden et al., 2003). Due to its targeting of the κ-light chain, PpL may, unlike SpA and SpG, bind to any class of antibody, provided it contains said κ-light chain. This versatility means Protein L can be applied, for example, to the isolation and purification of IgG, IgM, and IgA (Nilson et al., 1993). As with Protein A and Protein G, biochemical applications of Protein G are amenable to benefiting from the present invention. The aforementioned domains of Protein L are encompassed by the term "protein L" when used herein. Accordingly, a preferred protein domain comprised by the polypeptide of the present invention is a domain from Protein G as described herein.

In addition to polymerisation, numerous point mutations have further improved the function of SpA binding domains. One particularly relevant mutant is the so-called Z-domain, which was generated from introducing a G29A point mutation to the B-domain. This mutation eliminates the interaction with the Fab domain of IgG and reduces the acidity required for elution (Ghose et al., 2005; Nilsson et al., 1987). The H18S mutation of the Z-domain resulted in a further increase in the pH value required, and also positively influenced the binding capacity (Pabst et al., 2014). Bjorkman et al (2014) disclosed a mutation (P57I) in their patent that intended to improve the binding capacity of the ligand by removing a proline after helix 3, thus increasing flexibility. All of these point mutations are encompassed by the term "protein A" when used herein. A preferred Z domain is described herein and shown in SEQ ID NO 7. A preferred minimalized Z domain is described herein and shown in SEQ ID NO 8.

Attempts to enhance binding capacity of polymerized Protein A subunits have also been made by adapting the linkers used to join them. However, in contrast to the present invention, flexible amino acid linkers were used in the prior art, but not rigid amino acid linkers. Indeed, as has been found in context with the present invention, key to this invention is the impact that amino acid linker rigidity has on protein functionality. Rigid amino acid linkers ensure that a protein domain comprised by the polypeptide of the present invention is held in a fixed position relative to the other protein domains that the linker connects it to within the protein. Depending on the protein domain in question, this can ensure that unwanted inter-domain interactions do not occur, which may impede the functionality of one or both of those domains, or ensure stability of a desired inter-domain interaction by preventing movement that prevents that interaction. Either way, the effect of a rigid amino acid linker is to provide spatial stability, thereby promoting functional properties of the protein (Chen et al., 2013; Choi et al., 2019; George and Heringa, 2002).

The use of a peptide linker to join two or more domains together can result in the domains being joined in a manner that enhances their functionality, or even results in multi-functionality of the protein. That enhancement can, for example, result from preventing those domains from physically impeding one another, from ensuring linked domains retain flexibility so they can move independently relative to one another, or from facilitation of protein folding. The physicochemical properties of the linker greatly influence its functional effect within a fusion protein, so the precise nature of the linker must be carefully chosen so as to ensure that functional effect is optimized. One of the most important considerations to make when choosing a linker is between rigid and flexible linkers. Rigid linkers prevent movement, holding domains at fixed positions and orientations, whilst flexible linkers join domains so as to allow the domains to move relative to one another. The two most common class of rigid linkers are helical and non-helical linkers (Chen et al., 2013; George and Heringa, 2002). Dong et al (2015) used the hydrophilic, flexible sequence DDAKK (SEQ ID NO: 16) in different numbers of repeats to link protein A and protein G, with the longest linker providing the highest binding capacity. Linhult et al (2003) compared 3 linker sequences (VDANS (SEQ ID NO: 17), VDADS (SEQ ID NO: 18), GGGSG (SEQ ID NO: 19)) to dimerise a human serum albumin-binding protein domain from protein G. The VDADS (SEQ ID NO: 17) linker provided slightly higher binding capacities and increased base stability. According to the current state of research, there are no further attempts to improve IgG-binding domains by inserting or extending linker sequences, as is common practice with other fusion proteins, and the technical problem of limited binding capacity in fusion proteins comprised of binding proteins persists. However, the present invention tackled this issue and the inventors found that rigid amino acid linkers provide for an improvement in, e.g. dynamic binding capacity over flexible amino acid linkers which have been applied in the prior art. Of note, rigid amino acid linkers as described herein can not only be used to fuse immunoglobulin binding domains, but any other protein binding domain binding to a specific site on the surface of a target protein as described herein.

In the context of the present invention, the term "peptide linker" may be interchangeably used with the term "amino acid linker" and refers to a short amino acid sequence that connects two or more protein domains within both naturally occurring multi-domain proteins and artificial fusion proteins, e.g. a polypeptide of the present invention comprising protein domains. A peptide linker as applied in the polypeptide of the present invention is rigid. Preferred rigid peptide linkers are described herein.

In the context of the present invention, the term "rigid linker" which may be interchangeably used herein with the "rigid amino acid linker" or "rigid peptide linker" refers to any peptide linker that has limited or no flexibility, thus ensuring a stable spatial orientation and structure within a fusion protein comprising domains which are fused via a rigid linker, e.g. a polypeptide of the present invention. Accordingly, a "flexible linker" is any peptide linker that is not rigid, allowing movement of connected domains relative to one another. The present invention does not foresee the use of "flexible linkers" in a polypeptide of the present invention.

Rigid linkers exhibit preferably stiff structures by adopting α-helical structures or by, e.g. containing multiple Pro residues. Indeed, Pro is a unique amino acid with a cyclic side chain that causes a very restricted conformation. Furthermore, the lack of amide hydrogen on Pro typically prevents the formation of hydrogen bonds with other amino acids, and therefore reduces the interaction between the linkers and the protein domains. As a result, the inclusion of Pro residues might increase the stiffness and structural independence of the linkers. Furthermore, the length of the linkers can be easily adjusted by changing the copy number to achieve an optimal distance between protein domains. As a result, rigid linkers are chosen when the spatial separation of the protein domains is critical to preserve the stability or bioactivity of the various protein domains comprised by a fusion protein of the present invention. In this case, an example of a rigid linker is the alpha helix-forming linkers with the sequence of (EAAAK)n (SEQ ID NO: 11), which shows an approximately 80% helicity with n being 3. Helicity increase above 80%, if n is greater than 3, e.g., 4, 5, 6 or more. Generally, rigid linkers are characterized by high rigidity and stability, with intra-segment hydrogen bonds and a closely packed backbone. Therefore, the stiff a-helical linkers may act as simple rigid spacers between protein domains. Another type of rigid linkers, as explained above, has a Pro-rich sequence, (XP)n, with X designating any amino acid, preferably Ala, Lys, or Glu; with n being, e.g. 2, 4, 5, 6, 7 or more. Therefore, the presence of Pro in non-helical linkers can increase the stiffness, and allows for effective separation of the protein domains comprised by a polypeptide of the present invention as well.

In the case of artificially constructed proteins, e.g. a polypeptide of the present invention, the peptide linkers between protein domains should be carefully selected to ensure that the linker suits the needs of the construct. For example, when constructing a polypeptide of the present invention that comprises protein binding domains, rigid inter-domain linkers can prevent unwanted interaction between binding domains, ensuring that the binding domains are free to bind the desired target protein. Advances in the understanding of how linker amino acid composition determines those properties means that rational linker design or selection is now possible thanks to the present invention. The secondary structure adopted by the linker is often indicative of its properties, with helical, non-helical, β-stranded, or coil/bend structures commonplace amongst natural linkers. Helical structures, namely α-helices, confer inherent rigidity and stability whilst non-helical linkers tending to require proline-rich sequences in order to achieve similar rigidity (Chen et al., 2013; George and Heringa, 2002).

In another preferred embodiment of the invention, the rigid amino acid linker is non-helical. In the context of the present invention, a "non-helical rigid amino acid linker" refers to any rigid amino acid linker that does not contain an alpha helix within its secondary structure. Non-helical linkers comprise 51% of natural linkers identified in a comprehensive database of protein structures. These linkers lack an inherently rigid secondary structure, deriving their rigidity instead from the high number of proline residues they often contain, which appear in non-helical linkers 1.816 times more frequently than expected by chance (George and Heringa, 2002). This is due to the unique properties of proline, which as the only cyclic amino acid lacks an amide hydrogen to donate in hydrogen bonding, thus preventing it from participating in either α-helices or β-sheets. Additionally, the presence of a cyclic ring structure, wherein the side-chain loops back to bond to the amino group, results in highly restricted rotational flexibility (Ho et al., 2005).

In the context of the present invention, "a-helix" refers to a common structural motif found in the secondary structure of polypeptides, characterized by a right-handed coiled configuration stabilized by hydrogen bonds between the backbone amides of every fourth amino acid.

Accordingly, rigid non-helical linkers can be used when constructing multi-domain proteins in which it is important that the different domains do not interact with one another or when optimal protein function occurs when different domains are held at a fixed distance from one another. This is known to improve the binding capacity of, e.g. Protein A fusion proteins. Rigid non-helical linkers can be readily adapted to provide an optimal inter-domain distance by simply increasing the number of sequence repeats in the amino acid chain. The modular, linear nature of rigid non-helical linkers allows for readily predictable inter-domain distances to be created. Non-helical rigid linkers are generally not sequence specific beyond a high proline density (Amet et al., 2009; Chen et al., 2013; Maeda et al., 1997). Flexible non-helical linkers do not adopt any secondary structure and are typically composed of small, non-polar amino acids, such as glycine, and polar amino acids, such as serine and threonine. The small sizes of these amino acids allows for flexibility and the incorporation of polar amino acids results in hydrogen bonding with surrounding water molecules, thus preventing such interactions occurring between the linker and the protein domains themselves (Chen et al., 2013). Flexible linkers are employed when multi-domain proteins require conformational flexibility and/or independent functionality of heterologous protein domains, for example (Patel et al., 2020; van Rosmalen et al., 2017).

"Non-polar amino acids", as used herein, refers to the amino acids glycine, alanine, valine, leucine, isoleucine, proline, phenylalanine, methionine, and tryptophan. "Polar amino acids", as used herein, refers to the amino acids serine, cysteine, threonine, tyrosine, asparagine, and glutamine.

Specifically, in preferred embodiments the non-helical rigid amino acid linker comprises an amino acid sequence of 6 to 21 amino acids in length. Preferably, the amino acid sequence of 6 to 21 amino acids in length is a proline-rich amino acid sequence. More preferably, the non-helical rigid amino acid linker comprises the amino acid sequence (XP)2+n, wherein X is any amino acid and n is 0 to 8. More preferably still, the non-helical rigid amino acid linker comprises the amino acid sequence (XP)n, wherein X is any amino acid, except for D, E, K, or R, wherein, the n in the non-helical rigid amino acid linker (XP)n is preferably 2 to 10. A preferred linker is GSAPAPAPAPASG (SEQ ID NO: 20). It is to mention that the non-helical rigid amino acid linker with the proline-rich amino acid sequence comprises the (XP)2+n or (XP)n motif and additional amino acids so that a preferred length of minimal 6 amino acids is reached.

In a further preferred embodiments, the non-helical rigid amino acid linker comprises an amino acid sequence of 6 to 50 amino acids. Preferably, the amino acid sequence of 6 to 21 amino acids in length is a proline-rich amino acid sequence. More preferably, the non-helical rigid amino acid linker comprises the amino acid sequence (XP)2+n, wherein X is any amino acid and n is 0 to 20. More preferably still, the X in the non-helical rigid amino acid linker comprises the amino acid sequence (XP)n, wherein X is any amino acid, except for D, E, K, or R, wherein, the n in the non-helical rigid amino acid linker (XP)n is preferably 2 to 20. It is to mention that the non-helical rigid amino acid linker with the proline-rich amino acid sequence comprises the (XP)2+n or (XP)n motif and additional amino acids so that a preferred length of minimal 6 amino acids is reached.

In a preferred embodiment of the invention, the non-helical rigid amino acid linker (XP)n or (XP)2+n is flanked N-terminal and/or C-terminal by the amino acid sequence (GxSy)z. Preferably, the x in the amino acid sequence (GxSy)z is 1 to 10, y in the amino acid sequence (GxSy)z is 1 to 10, z in the amino acid sequence (GxSy)z is 1 to 10.

Alternatively, but preferred in the context of the present invention, the rigid amino acid linker is helical. In the context of the present invention, this refers to any rigid amino acid linker that contains an a-helical secondary structure. Almost 40% of natural linkers contain $\alpha$-helices. Due to extensive hydrogen bonding and the density of the helix backbone, $\alpha$-helix linkers provide rigid spacing between adjacent protein domains (George and Heringa, 2002). Since the development of a linker with an $\alpha$-helix in 2001 (Arai et al., 2001), helical linkers have been commonly applied to the construction of recombinant multi-domain protein constructs (Chen et al., 2013). The structural constraints imposed by an $\alpha$-helix allow for predictable, rigid structures to be formed when engineering novel fusion proteins. Correct folding of an unbroken helix that links two protein domains allows the relative orientation that those two domains are held at to be readily predicted. As with rigid non-helical linkers, helical linkers can be used in a modular fashion to create a desired inter-domain distance and/or orientation (Chen et al., 2013; Lai et al., 2015). The most commonly used helical linker motif employed in fusion protein engineering is the sequence A(EAAAK)$_n$ (SEQ ID NO: 10). This motif was first identified in 2001, with the helix forming peptide AEAAAKEAAAKEAAAKA (SEQ ID NO: 12) demonstrated to be highly effective as a linker that can be used to separate functional domains in a fusion protein. The stability of this motif derives from hydrogen bonding between glutamate and lysine residues (Arai et al., 2001).

In a preferred embodiment of the invention, the helical amino acid linker comprises an amino acid sequence which locally forms a single a-helical domain. Preferably, the helical amino acid linker comprises an amino acid sequence of 10 to 30 amino acids in length. Preferably, the amino acid sequence of 10 to 30 amino acids in length comprises the amino acids E, K and/or R. Preferably, the amino acid sequence of 10 to 30 amino acids in length comprises (EAAAK)n (SEQ ID NO: 11). Preferably, the n in the amino acid sequence (EAAAK)n (SEQ ID NO: 11) is 1 to 20.

Preferred amino acid linkers used in the polypeptide of the present invention to link protein domains are shown in SEQ ID NOs: 10 to 15 and 20.

In a preferred embodiment of the invention, the protein domains comprised by the polypeptide of the present invention are PDZ domains, PKD domains, lectins, peptide aptamers, IgG binding domains, IgA binding domains or IgM binding domains. All of these domains are examples of binding domains.

In the context of the present invention, "PDZ domains" refers to PDZ (PSD-95, DiscsLarge, ZO1) domains which function in nature as protein binding domains within scaffold and membrane-associated proteins. They comprise about 90 residues and make specific, high affinity interactions with complementary C-terminal peptide sequences, with other PDZ domains, and with phospholipids (Walkup and Kennedy (2014).

In the context of the present invention, "PKD domains" refers to Polycystic Kidney Disease domains, a protein domain found in various proteins that facilitates specific protein-protein interactions and/or protein-carbohydrate interactions. These domains often participate in cell-cell or cell-matrix adhesion processes by binding to other proteins. A "PKD domain" contains an Ig-like fold with a beta-sandwich of seven strands in two sheets. Said term also includes a modified PKD domain, a fragment of a PKD domain, preferably it is capable of binding an adeno associated virus (AAV). Preferred PKD domains in the context of the present invention can be taken from the Polycystic kidney disease-associated protein PKD1 from human having Uniprot accession number Q15142. A preferred PKD1 protein is shown in SEQ ID NO: 22. The skilled person is able to identify and then take PKD domains from proteins known to have PKD domains, e.g. from the PKD1 protein from human having Uniprot accession number Q15142 or from the PKD1 protein shown in SEQ ID NO: 22.

In the context of the present invention, "lectins" refers to a protein that contain binding domains that with high specificity toward carbohydrates. Lectins will often contain multiple binding domains which display selectivity toward specific carbohydrate structures e.g. specific mono- or oligosaccharides. As with other binding domain comprising proteins, lectins can be enhanced through the use of rigid linkers to increase stability and binding capacity. Lectins can be applied to the isolation of glycoproteins through recognition and binding of attached carbohydrates (Chettri et al., 2021; Raposo et al., 2021; Vijayan and Chandra, 1999).

In the context of the present invention, "peptide aptamers" refers to proteins that are selected to bind to specific sites on their target molecules. Peptide aptamers comprise 5-30 amino acids, preferably 20-30 amino acid residues long sequences, embedded as a loop within a stable protein scaffold. Peptide aptamers comprise one or more short variable peptide domains. The most common peptide aptamer selection system is the yeast two-hybrid system. Peptide aptamers may be selected from combinatorial peptide libraries constructed by phage display and other surface display technologies such as mRNA display, ribosome display, bacterial display and yeast display. These experimental procedures are also known as biopanning. All the peptides panned from combinatorial peptide libraries have been stored in the MimoDB database (Huang et al., 2012). Peptide aptamers and the way to generate them are known in the art (see Reverdatto et al., 2015)

In a preferred embodiment of the invention, the IgG binding domain comprised by the polypeptide of the present invention comprises an amino acid sequence which locally forms at least one alpha-helix. This corresponds to the known structure of the SpA IgG binding domain, which folds into a 3 alpha-helix bundle structure. Preferably, the linker is located after the amino acid sequence forming the last alpha-helix of each IgG-binding domain and before the amino acid sequence forming the first alpha-helix of the further IgG-binding domain. Accordingly, this will act to provide a fixed distance between each IgG-binding domain, thus allowing the peptide of the present invention to improve IgG binding capacity. In accordance with the structure of the SpA IgG binding domain as known to one skilled in the art, the IgG binding domain preferably comprises an amino acid sequence locally forming at least two alpha-helices, preferably three alpha-helices. More preferably, the alpha-helix, referred to herein, is formed by an amino acid sequence of 10 to 14 amino acids in length. Additionally, in a preferred embodiment of the invention the IgG binding domain preferably comprises an amino acid sequence further locally forming one or more beta-pleated sheets.

It has been demonstrated previously that increasing the number of binding domains present in a fusion protein can enhance binding capacity. For example, fusion proteins comprising the B or C domains of SpA typically contain 4 to 6 copies of said domain (Pabst et al., 2018). As shown and exemplified herein, and supported by prior art (Freiherr von Roman and Berensmeier, 2014), increasing that number further can lead to even higher IgG binding capacity. Accordingly, in a preferred embodiment of the invention, the polypeptide of the present invention comprises 2, 3, 4, 5, 6, 7, 8, 9, 10 or more IgG binding domains. However, as explained herein, the present invention is an improvement over e.g. the fusion proteins of the prior art, because the inventors recognized that a rigid linker between protein domains contributes to an increase in the binding capacity of the fusion protein, here the polypeptide of the present invention.

In a preferred embodiment of the IgG binding domains comprised by the polypeptide of the present invention, a naturally-occurring amino acid sequence between the last alpha-helix of an IgG-binding domain and the first alpha-helix of a further IgG-binding domain is present or absent.

In a preferred embodiment of the IgG binding domains comprised by the polypeptide of the present invention, at least one of the IgG-binding domains contains between two alpha helices an EF-hand loop (Kanje et al., 2020) and see also WO2018/046475.

Preferably, the IgG binding domain comprised by the polypeptide of the present invention is the A-domain of protein A, B-domain of protein A, C-domain of protein A, D-domain of protein A, E-domain of protein A, the Z-domain of protein A, an IgG binding domain of protein G, or an IgG binding domain of protein L. These domains comprise the 5 endogenous IgG-binding domains found in native SpA, along with the engineered variant of the B-domain, the Z-domain. In addition, the IgG binding-domain of Protein G, also found in *Streptococcus* bacteria, and the IgG binding-domain of Protein L, found in *Peptostreptococcus magnus* and which targets the κ-light chain of antibodies, are also viable binding domains to use in preferred embodiments of the polypeptide of the present invention.

In a preferred embodiment of the present invention, the A-domain of protein A comprised by the polypeptide of the present invention has an amino acid sequence which is at least 70%, 80%, 90% or 95% identical to the amino acid sequence shown in SEQ ID NO: 9, more preferably the A-domain of protein A has the amino acid sequence shown in SEQ ID NO: 2

In a preferred embodiment of the present invention, the B-domain of protein A comprised by the polypeptide of the present invention has an amino acid sequence which is at least 70%, 80%, 90% or 95% identical to the amino acid sequence shown in SEQ ID NO: 9, more preferably the B-domain of protein A has the amino acid sequence shown in SEQ ID NO: 3.

In a preferred embodiment of the present invention, the C-domain of protein A comprised by the polypeptide of the present invention has an amino acid sequence which is at least 70%, 80%, 90% or 95% identical to the amino acid sequence shown in SEQ ID NO: 9, more preferably the C-domain of protein A has the amino acid sequence shown in SEQ ID NO: 4.

In a preferred embodiment of the present invention, the D-domain of protein A comprised by the polypeptide of the present invention has an amino acid sequence which is at least 70%, 80%, 90% or 95% identical to the amino acid sequence shown in SEQ ID NO: 9, more preferably the D-domain of protein A has the amino acid sequence shown in SEQ ID NO: 5.

In a preferred embodiment of the present invention, the E-domain of protein A comprised by the polypeptide of the present invention has an amino acid sequence which is at least 70%, 80%, 90% or 95% identical to the amino acid sequence shown in SEQ ID NO: 9, more preferably the E-domain of protein A has the amino acid sequence shown in SEQ ID NO: 6.

In a preferred embodiment of the present invention, the Z-domain of protein A comprised by the polypeptide of the present invention has an amino acid sequence which is at least 70%, 80%, 90% or 95% identical to the amino acid sequence shown in SEQ ID NO: 9, more preferably the Z-domain of protein A has the amino acid sequence shown in SEQ ID NO: 7.

In a preferred embodiment of the present invention, the Z-domain of protein A is a minimalized Z domain having the amino acid sequence shown in SEQ ID NO: 8. A preferred consensus sequence for the amino acid sequences encoding the A, B, C, D, E, and Z domains is shown in SEQ ID NO: 9, wherein X1: N or H; X2: M or L, X3: S or A.

The level of identity between two or more sequences (e.g., nucleic acid sequences or amino acid sequences) can be easily determined by methods known in the art, e.g., by BLAST analysis. Generally, in context with the present invention, if two sequences (e.g., polynucleotide sequences or amino acid sequences) to be compared by, e.g., sequence comparisons differ in identity, then the term "identity" may refer to the shorter sequence and that part of the longer sequence that matches said shorter sequence. Therefore, when the sequences which are compared do not have the same length, the degree of identity may preferably either refer to the percentage of nucleotide residues in the shorter sequence which are identical to nucleotide residues in the longer sequence or to the percentage of nucleotides in the longer sequence which are identical to nucleotide sequence in the shorter sequence. In this context, the skilled person is readily in the position to determine that part of a longer sequence that matches the shorter sequence. Furthermore, as used herein, identity levels of nucleic acid sequences or amino acid sequences may refer to the entire length of the respective sequence and is preferably assessed pair-wise, wherein each gap is to be counted as one mismatch. These definitions for sequence comparisons (e.g., establishment of "identity" values) are to be applied for all sequences described and disclosed herein.

Moreover, the term "identity" as used herein means that there is a functional and/or structural equivalence between the corresponding sequences. Nucleic acid/amino acid sequences having the given identity levels to the herein-described particular nucleic acid/amino acid sequences may represent derivatives/variants of these sequences which, preferably, have the same biological function. They may be either naturally occurring variations, for instance sequences from other varieties, species, etc., or mutations, and said mutations may have formed naturally or may have been produced by deliberate mutagenesis. Furthermore, the variations may be synthetically produced sequences. The variants may be naturally occurring variants or synthetically produced variants or variants produced by recombinant DNA techniques.

As used herein, unless specifically defined otherwise, the term "nucleic acid" or "nucleic acid molecule" is used synonymously with "oligonucleotide", "nucleic acid strand", or the like, and means a polymer comprising one, two, or more nucleotides, arranged in single- or double stranded nucleotide chains. Generally, as used herein, the terms "polynucleotide", "nucleic acid" and "nucleic acid molecule" are to be construed synonymously. Generally, nucleic acid molecules may comprise inter alia DNA molecules, RNA molecules, oligonucleotide thiophosphates, substituted ribooligonucleotides or PNA molecules. Furthermore, the term "nucleic acid molecule" may refer to DNA or RNA or hybrids thereof or any modification thereof that is known in the art (see, e.g., US 5525711, US 4711955, US 5792608 or EP 302175 for examples of modifications). The nucleic acid molecule may be single- or double- stranded, linear or circular, natural or synthetic, and without any size limitation. For instance, the nucleic acid molecule may be genomic DNA, cDNA, mitochondrial DNA, mRNA, antisense RNA, ribozymal RNA or a DNA encoding such RNAs or chimeroplasts. Said nucleic acid molecule may be in the form of a vector, plasmid, cosmid, or of viral DNA or RNA. Also described herein are nucleic acid molecules which are complementary to the nucleic acid molecules described above and nucleic acid molecules which are able to hybridize to nucleic acid molecules described herein. A nucleic acid molecule described herein may also be a fragment of the nucleic acid molecules in the context of the present invention. Particularly, such a fragment is a functional fragment. Examples of such functional fragments are nucleic acid molecules which can serve as primers.

In a preferred embodiment of the invention, the carrier material to which the polypeptide of the present invention is coupled is a particle, bead, resin, membrane, solid support or tag. Carrier materials are the physical structures to which binding proteins are immobilized when performing affinity chromatography or similar biochemical methods. The composition of the carrier material and the structure of the material (e.g. beads vs layer) must be altered depending on the desired use of the binding protein and both significantly impact on the performance of the binding assay. Common carrier materials include agarose, sepharose, glass, and sugar- or acrylamide-based polymer resins. The binding protein is covalently attached to the carrier material, also termed an inert matrix or solid support, and this allows the binding protein to bind to the target protein and remove it from solution. Binding capacity can be increased through the use of carrier materials that exhibit high surface area to volume ratio, such as agarose beads. Binding proteins may be attached to the carrier material in a quasi-random manner, or immobilized in a site-directed fashion. Random immobilization can result in coupling between the binding proteins and the carrier material in a manner that results in the binding domain of the immobilized protein being blocked by either the carrier surface itself or other binding proteins. Furthermore, binding proteins can be attached as either monomers or as polymers, with further improvement of binding capacity possible through the use of linkers to ensure adequate spacing between adjacent binding proteins to prevent steric hinderance of target protein binding (Chen et al., 2013).

Any suitable technique may be used to attach a ligand to a support, such as a solid support, utilizing methods well-known in the art and described herein. For instance, the ligand may be coupled to the support using conventional techniques involving amino and/or carboxy groups present in the ligand, with common coupling reagents including bisepoxides, epichlorohydrin, CNBr, and N-hydroxysuccinimide (NHS). In some cases, a spacer is introduced between the support and the ligand to enhance ligand availability and facilitate chemical coupling. Alternatively, noncovalent bonding methods, such as physical or bispecific adsorption, may be employed. In various embodiments, the ligand can be attached to a solid support, such as chromatography resin, at multiple sites to create a chromatography matrix. Protein ligands may be coupled to a solid support via active groups on the support or ligand, such as hydroxyl, thiol, epoxide, amino, carbonyl, or carboxylic acid groups, using chemistries like cyanogen bromide (CNBr), N-hydroxysuccinimide ester, epoxy activation, and reductive amination. Thiol-directed protein coupling, as described by (Ljungquist et al., 1989), has been used to attach SpA to a solid support. Since wild-type SpA lacks thiol groups, attachment is achieved by recombinantly inserting a thiol-containing cysteine at the C-terminus of SpA, as outlined in U.S. Patent No. 6399750.

Furthermore, the present invention provides a method for producing the polypeptide of the present invention, comprising coupling the polypeptide of the present invention to a carrier material.

The polypeptide of the present invention may be produced by expressing a nucleotide sequence encoding the polypeptide of the present invention in a host cell or by synthesizing said polypeptide, and coupling the polypeptide to a carrier material. Any suitable host cell can be used to produce the polypeptide. A large number of suitable methods exist in the art to produce the polypeptide of the present invention in a host cell. Conveniently, the produced protein is harvested from the culture medium, lysates of the cultured host cell, or from isolated (biological) membranes by established techniques. For example, the nucleotide sequence encoding the polypeptide of the present invention can be synthesized by PCR and inserted into an expression vector. Subsequently a host cell may be transformed or transfected with the expression vector. Thereafter, the cell is cultured to produce/express the polypeptide of the present invention, which may then be isolated and purified. For example, the polypeptide may be recovered from the host cell and/or culture medium by conventional procedures including, but not limited to, cell lysis, breaking up host cells, centrifugation, filtration, ultra-filtration, extraction or precipitation. Purification may be performed by a variety of procedures known in the art including, but not limited to, chromatography (e.g. ion exchange, affinity, hydrophobic, chromatofocusing, and size exclusion), electrophoretic procedures (e.g., preparative isoelectric focusing), differential solubility (e.g. ammonium sulfate precipitation) or extraction

The present invention additionally provides a kit comprising the polypeptide of the present invention and a carrier material. Kits may further comprise buffers for carrying out reactions and/or reagents for coupling a carrier material to the polypeptide of the present invention. Namely, the kit may comprise the polypeptide of the present invention without being coupled to a carrier. Accordingly, the kit, as described above, may comprise buffers for carrying out reactions and/or reagents for coupling a carrier material to the polypeptide of the present invention. Said carrier material may be a particle, bead, resin, membrane, solid support or tag

The present invention further provides a method for producing a purified target protein comprising eluting said target protein bound to the polypeptide of the present invention. By way of example, the current state of the art regarding elution of bound target proteins during, but not limited to, affinity chromatography can be found in WO 2011/073389. In the context of the present invention and as used herein, "elution" refers to a process comprising extracting a substance from a mixture, typically by washing it out with a solvent during chromatography.

Of course, before a target protein can be eluted from the polypeptide of the present invention, it has to be bound by the polypeptide of the present invention. Accordingly, the polypeptide of the present invention and a target protein are brought into contact under conditions allowing the target protein to bind to the polypeptide of the present invention and vice versa, i.e., the polypeptide of the present invention binds to the target protein as described herein.

The present invention further provides the use of the polypeptide of the present invention for the production of a purified target protein. All embodiments described herein in the context of the method for producing a purified target protein apply to said use, mutatis mutandis.

It is to be noted that in case of any definition given herein, the respective definition of a term, phrase, and/or abbreviation applies vice versa throughout the specification. Furthermore, all definition given herein are intended to encompass all grammatical forms.

The following sequences are referred to herein:

SEQ ID NO: 1 (Protein A))

AAQHDEAQQNAFYQVLNMPNLNADQRNGFIQSLKDDPSQSANVLGEAKKLNESQAPKADNNFNKEQQNAFYEI
LNMPNLNEEQRNGFIQSLKDDPSQSANLLSEAKKLNESQAPKADNKFNKEQQNAFYEILHLPNLNEEQRNGFIQSL
KDDPSQSANLLAEAKKLNDAQAPKADNKFNKEQQNAFYEILHLPNLTEEQRNGFIQSLKDDPSVSKEILAEAKKLND
AQAPKEEDNNKPGKEDGNKPGKEDGN

SEQ ID NO: 2 (Domain A of Protein A)
ADNNFNKEQQNAFYEILNMPNLNEEQRNGFIQSLKDDPSQSANLLSEAKKLNESQAPK

SEQ ID NO: 3 (Domain B of Protein A)
ADN KFN KEQQNAFYEI LH LPN LN EEQRNG FIQSLKDDPSQSAN LLAEAKKLN DAQAPK

SEQ ID NO: 4 (Domain C of Protein A)
ADNKFNKEQQNAFYEILHLPNLTEEQRNGFIQSLKDDPSVSKEILAEAKKLNDAQAPK

SEQ ID NO: 5 (Domain D of Protein A)
ADNNFNKDQQSAFYEILNMPNLNEAQRNGFIQSLKDDPSQSTNVLGEAKKLNESQAPK

SEQ ID NO: 6 (Domain E of Protein A)
ADAQHDEAQQNAFYQVLNMPNLNADQRNGFIQSLKDDPSQSANVLGEAQKLNDSQAPK

SEQ ID NO: 7 (Domain Z of Protein A)
VDNKFNKEQQNAFYEILHLPNLNEEQRNAFIQSLKDDPSQSANLLAEAKKLNDAQAPK

SEQ ID NO: 8 (Minimalized domain Z of Protein A)
FNMQQQRRFYEALHDPNLNEEQRNAKIKSIRDD

SEQ ID NO: 9 (Consensus sequence of Protein A domains)

ADNKFNKEQQNAFYEILX1X2PNLNEEQRNGFIQSLKDDPSQSANLLAEAKKLNDX3QAPK

X1: N or H

X2: M or L

X3: S or A

SEQ ID NO: 10 (helical linker motif)
AEAAAK

SEQ ID NO: 11 (helical rigid linker)
EAAAK

SEQ ID NO: 12 (helix forming peptide linker)
AEAAAKEAAAKEAAAKA

SEQ ID NO: 13 (linker)
QAPKGSAPAPAPAPASGARADNKFHK

SEQ ID NO: 14 (linker)
QAPKILEADNKFHK

SEQ ID NO:15 (linker)
QAPKGGGGSGGGGSARADNKFHK

SEQ ID NO: 16 (linker)
DDAKK

SEQ ID NO: 17 (linker)
VDANS

SEQ ID NO: 18 (linker)
VDADS

SEQ ID NO: 19 (linker)
GGGSG

SEQ ID NO: 20 (linker)
GSAPAPAPAPASG

SEQ ID NO: 21 (linker)
GGGGSGGGGS

SEQ ID NO: 22 (human PKD1 protein)

MPPAAPARLALALGLGLWLGALAGGPGRGCGPCEPPCLCGPAPGAACRVNCSGRGLRTLGPALRIPADATALTAM
AKADGDVKKYGSKRGALALTHGHSLLRDVSHNLLRALDVGLLANLSALAELDISNNKISTLEEGIFANLFNLSEINLSG
NPFECDCGLAWLPRWAEEQQVRVVQPEAATCAGPGSLAGQPLLGIPLLDSGCGATLVGPHGPLASGQLAAFHIAA
PLPVTATRWDFGDGSAEVDAAGPAASHRYVLPGRYHVTAVLALGAGSALLGTDVQVEAAPAALELVCPSSVQSDE
SLDLSIQNRGGSGLEAAYSIVALGEEPARAVHPLCPSDTEIFPGNGHCYRLVVEKAAWLQAQEQCQAWAGAALAM
VDSPAVQRFLVSRVTRSLDVWIGFSTVQGVEVGPAPQGEAFSLESCQNWLPGEPHPATAEHCVRLGPTGWCNTD
LCSAPHSYVCELQPGGPVQDAENLLVGAPSGDLQGPLTPLAQQDGLSAPHEPVEAGMGSVELRRPPAHQVMVFP
GLRLSREAFLTTAEFGTQELRRPAQLRLQVYRLLSTAGTPENGSEPESRSPDNRTQLAPACMPGGRWCPGANICLPL
DASCHPQACANGCTEGEHVVDVVVENSASRANLSLRVTAEEPICGLRATPSPEARVLQGVLVRYSPVVEAGSDMVF
RWTINDKQSLTFQNVVFNVIYQSAAVFKLSLTASNHVSNVTVNYNVTVERMNRMQGLQVSTVPAVLSPNATLALT
AGVLVDSAVEVAFLWTFGDGEQALHQFQPPYNESFPVPDPSVAQVLVEHNVMHTYAAPGEYLLTVLASNAFENRT
QQVPVSVRASLPSVAVGVSDGVLSQPAANHTYASRGTYHVRLEVNNTVSGAAAQADVRVFEELRGLSVDMSLAVE
QGAPVVVSAAVQTGDNITWTFDMGDGTVLSGPEATVEHVYLRAQNCTVTVGAASPAGHLARSLHVLVFVLEVLR
VEPAACIPTQPDARLTAYVTGNPAHYLFDWTFGDGSSNTTVRGCPTVTHNFTRSGTFPLALVLSSRVNRAHYFTSIC
VEPEVGNVTLQPERQFVQLGDEAWLVACAWPPFPYRYTWDFGTEEAAPTRARGPEVTFIYRDPGSYLVTVTASNN
ISAANDSALVEVQEPVLVTSIKVNGSLGLELQQPYLFSAVGRGRPASYLWDLGDGGWLEGPEVTHAYNSTGDFTGP
GPGRQRQRLLAHRLEAGTYHVQLRATNMLGSAWADCTMDFVEPVGWLMVAASPNPAAVNKSVTLSAELAGGS
GVVYTWSLEEGLSWETSEPFTTHSFPTPGLHLVTMTAGNPLGSANATVEVDVQVPVSGLSIRASEPGGSFVAAGSS
VPFWGQLATGTNVSWCWAVPGGSSKRGPHVTMVFPDAGTFSIRLNASNAVSWVSATYNLTAEEPIVGLVLWASS
KVVAPGQLVHFQILLAAGSAVTFRLQDTDEPRAEHSYLRPGDYRVQVNASNLVSFFVAQATVTVQVLACREPEVDV
VLPLQVLMRRSQRNYLEAHVDLRDCVTYQTEYRWEVYRTASCQRPGRPARVALPGVDVSRPRLVLPRLALPVGHY
CFVFVVSFGDTPLTQSIQANVTVAPERLVPIIEGGSYRVWSDTRDLVLDGSESYDPNLEDGDQTPLSFHWACVASTQ
REAGGCALNFGPRGSSTVTIPRERLAAGVEYTFSLTVWKAGRKEEATNQTVLIRSGRVPIVSLECVSCKAQAVYEVSR
SSYVYLEGRCLNCSSGSKRGRWAARTFSNKTLVLDETTTSTGSAGMRLVLRRGVLRDGEGYTFTLTVLGRSGEEEGC

ASIRLSPNRPPLGGSCRLFPLGAVHALTTKVHFECTGWHDAEDAGAPLVYALLLRRCRQGHCEEFCVYKGSLSSYGA
VLPPGFRPHFEVGLAVVVQDQLGAAVVALNRSLAITLPEPNGSATGLTVWLHGLTASVLPGLLRQADPQHVIEYSLA
LVTVLNEHRAQIRKNITETLVSLRVHTVDDIQQIAAALAQCMGPSRELVCRSCLKQTLHKLEAMMLILQAETTAGTV
TPTAIGDSILNITGDLIHLASSDVRAPQPSELGAESPSRMVASQAYNLTSALMRILMRSRVLNEEPLTLAGEEIVAQGK
RSDPRSLLCYGGAPGPGCHFSIPEAFSGALANLSDVVQLIFLVDSNPFPFGYISNYTVSTKVASMAFQTQAGAQIPIE
RLASERAITVKVPNNSDWAARGHRSSANSANSVVVQPQASVGAVVTLDSSNPAAGLHLQLNYTLLDGHYLSEEPEP
YLAVYLHSEPRPNEHNCSASRRIRPESLQGADHRPYTFFISPGSRDPAGSYHLNLSSHFRWSALQVSVGLYTSLCQYF
SEEDMVWRTEGLLPLEETSPRQAVCLTRHLTAFGASLFVPPSHVRFVFPEPTADVNYIVMLTCAVCLVTYMVMAAI
LHKLDQLDASRGRAIPFCGQRGRFKYEILVKTGWGRGSGTTAHVGIMLYGVDSRSGHRHLDGDRAFHRNSLDIFRI
ATPHSLGSVWKIRVWHDNKGLSPAWFLQHVIVRDLQTARSAFFLVNDWLSVETEANGGLVEKEVLAASDAALLRF
RRLLVAELQRGFFDKHIWLSIWDRPPRSRFTRIQRATCCVLLICLFLGANAVWYGAVGDSAYSTGHVSRLSPLSVDTV
AVGLVSSVVVYPVYLAILFLFRMSRSKVAGSPSPTPAGQQVLDIDSCLDSSVLDSSFLTFSGLHAEAFVGQMKSDLFL
DDSKSLVCWPSGEGTLSWPDLLSDPSIVGSNLRQLARGQAGHGLGPEEDGFSLASPYSPAKSFSASDEDLIQQVLAE
GVSSPAPTQDTHMETDLLSSLSSTPGEKTETLALQRLGELGPPSPGLNWEQPQAARLSRTGLVEGLRKRLLPAWCAS
LAHGLSLLLVAVAVAVSGWVGASFPPGVSVAWLLSSSASFLASFLGWEPLKVLLEALYFSLVAKRLHPDEDDTLVESP
AVTPVSARVPRVRPPHGFALFLAKEEARKVKRLHGMLRSLLVYMLFLLVTLLASYGDASCHGHAYRLQSAIKQELHS
RAFLAITRSEELWPWMAHVLLPYVHGNQSSPELGPPRLRQVRLQEALYPDPPGPRVHTCSAAGGFSTSDYDVGWE
SPHNGSGTWAYSAPDLLGAWSWGSCAVYDSGGYVQELGLSLEESRDRLRFLQLHNWLDNRSRAVFLELTRYSPAV
GLHAAVTLRLEFPAAGRALAALSVRPFALRRLSAGLSLPLLTSVCLLLFAVHFAVAEARTWHREGRWRVLRLGAWAR
WLLVALTAATALVRLAQLGAADRQWTRFVRGRPRRFTSFDQVAQLSSAARGLAASLLFLLLVKAAQQLRFVRQWS
VFGKTLCRALPELLGVTLGLVVLGVAYAQLAILLVSSCVDSLWSVAQALLVLCPGTGLSTLCPAESWHLSPLLCVGLW
ALRLWGALRLGAVILRWRYHALRGELYRPAWEPQDYEMVELFLRRLRLWMGLSKVKEFRHKVRFEGMEPLPSRSS
RGSKVSPDVPPPSAGSDASHPSTSSSQLDGLSVSLGRLGTRCEPEPSRLQAVFEALLTQFDRLNQATEDVYQLEQQL
HSLQGRRSSRAPAGSSRGPSPGLRPALPSRLARASRGVDLATGPSRTPLRAKNKVHPSST

SEQ ID NO: 23 (linker)
QAPKGSAPAPASGARADNKF

SEQ ID NO: 24 (linker)
GSAPAPASG

SEQ ID NO: 25 (linker)
QAPKGSAPAPAPAPAPASGARADNKF

SEQ ID NO: 26 (linker)
GSAPAPAPAPAPASG

SEQ ID NO: 27 (linker)
QAPKGSEAAAKEAAAKEAAAKSGARADNK

SEQ ID NO: 28 (linker)
GSEAAAKEAAAKEAAAKSG

SEQ ID NO: 29 (B3 domain of Protein L with point mutation)
KEKTPEEPKEEVTIKANLIYADGKTQTAEFKGTFEEATAEAYRYADLLAKEHGKYTVDVADKGYTLNIKFAG

SEQ ID NO: 30 (linker)
YTLNIKFAGKEKTPEEP

SEQ ID NO: 31 (linker)
YTLNIKFAGSAPAPAPAPASG GKEKTPEEP

The Figures show:

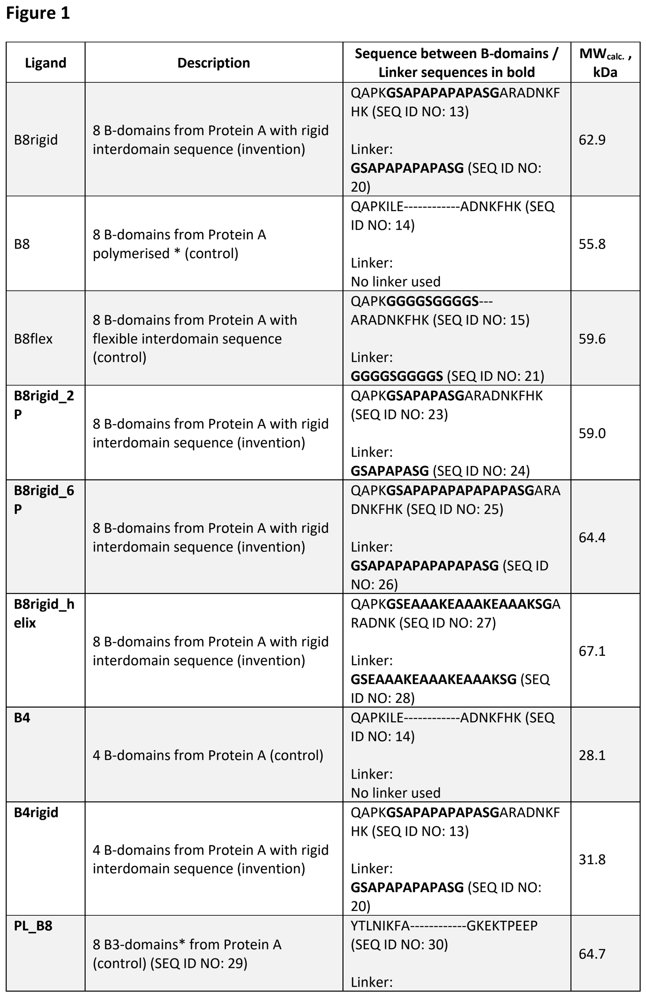

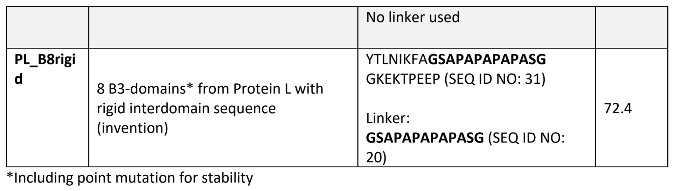

**Figure 1    Ligands used to evaluate effect of linker sequences on SpA B-domain binding properties** The ligands employed to evaluate the effect of different linker sequences on B-domain binding properties are summarized in a table. Shown is a description of the ligand, the linker sequence employed in the ligand, and the molecular weight of the ligand.

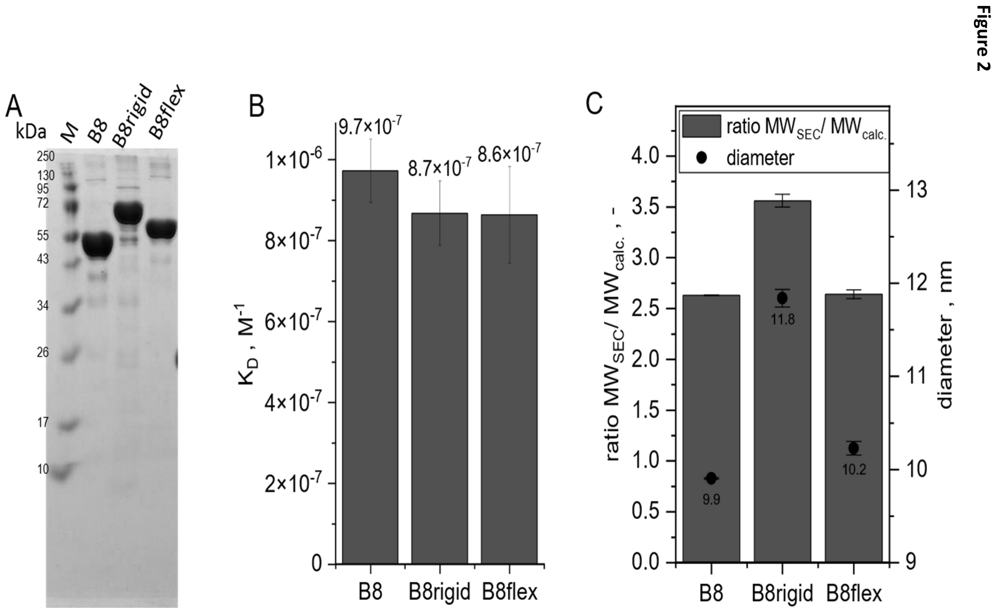

**Figure 2**    **Characterization of polymerised B-domains joined via different linker sequences** Proteins comprising 8 polymerised SpA B-domains joined via either native (B8), flexible (B8flex, control) or rigid (B8rigid, invention) amino acid linkers were characterized in terms of expression, ligand affinity constant, and globularity.

> **(A)** SDS-PAGE demonstrates successful expression and purification of all proteins evaluated, with molecular weights of said proteins matching the theoretical predictions of their MW, as seen in the Table shown in Figure 1. SDS-PAGE of the purified ligands shows, from right to left, marker NEB P7719 (M), B8, B8rigid, and B8flex.

> **(B)** Ligand affinity constant (kD) for IgG was evaluated through the use of surface plasmon resonance (SPR). The affinity for IgG remains unchanged by the addition of rigid or flexible sequences to the B8 polymer.

> **(C)** SEC-HPLC analysis shows that the proteins, particularly those with the rigid interdomain sequence B8rigid, exhibit a large $MW_{SEC}/MW_{calc}$ ratio, indicating they are more unfolded compared to globular standards. The ligands are similar in size to IgG (~12 nm), with B8rigid displaying the largest $MW_{sec}/MW_{calc}$ ratio while the quotient for the two controls is similar despite the different $MW_{calc}$. The ligand B8rigid is therefore more unfolded than the polymerised B8 wild type or the control with the additional flexible sequence, indicative of structural rigidity, as desired for the polypeptide of the present invention.

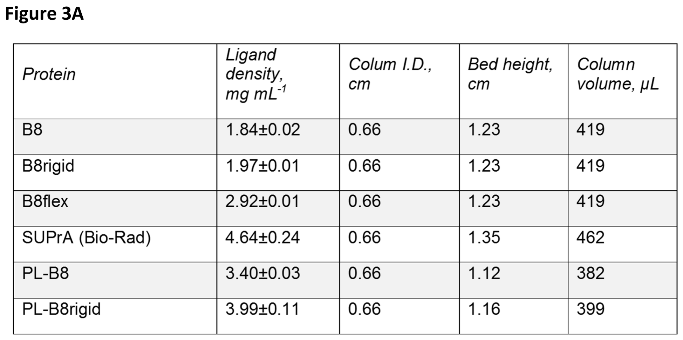

**Figure 3**    **B8 polymers show enhanced dynamic binding capacity when constructed using rigid linkers** B8 polymers were immobilized on an epoxy resin and their dynamic binding capacity (DBC) compared at two different residence times.

> **(A)** shows a table summarizing resin and column Information. This table provides details on the characteristics of the epoxy-activated resins and columns used in the dynamic binding capacity experiments, including resin types, column dimensions, and experimental conditions.

> **(B)** shows the measured dynamic binding capacities of B8, B8rigid, B8flex, and SUPrA, determined at a breakthrough concentration of 10% of the feed solution (DBC10%). Standard deviation from 2 individual measurements is shown for each experimental ligand, while the data for the SUPrA resin is based on one replicate. The ligand with the rigid interdomain sequence (B8rigid) demonstrates a significantly higher binding capacity, binding over 1.5 times more IgG than the control B8 ligand, despite similar ligand densities. The commercial resin based on recombinant protein A, SUPrA, exhibits a higher ligand density but performs worse than B8rigid.

> **(C)** shows the measured dynamic binding capacities of the Protein L-derived ligands PL_B8 and PL_B8rigid, determined at a breakthrough concentration of 10% of the feed solution (DBC10%) after subtraction of the non-binding lambda light chain IgG species at a single residence time of 2.1 min. The PL_B8rigid material shows a higher DBC10%. Even when taking into account the effect of the slightly different ligand densities, the beneficial effect remains evident.

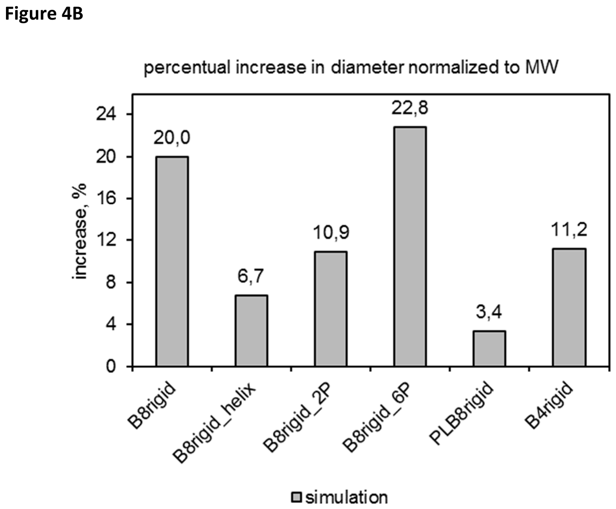

Figure 4    Prediction shows increase in the hydrodynamic properties for ligands incorporating the invented sequences

> **(A)** shows the predicted hydrodynamic diameters (based on the Stokes radii) derived from the procedure described under Example 4 compared with the experimentally derived values from Figure 2.

> **(B)** Normalizes the percentual increase in the diameter to the molecular weight of the respective ligand. The invented linker sequences lead to an overprortional increase in the hydrodynamic diameter that may have influence in the increase in the dynamic binding capacity of the binding partners of these ligands.

The present invention may also be characterized by the following items:

1. A polypeptide comprising protein domains binding to a specific site on the surface of a target protein, each protein

EP 4 729 534 A1

domain is translationally fused in its N- to C-terminal orientation to a further protein domain that is also in its N- to C-terminal orientation, via a rigid amino acid linker keeping a fixed distance between said protein domains and said rigid amino acid linker is also in its N- to C-terminal orientation, , wherein said polypeptide is coupled to a carrier material, wherein said carrier material is a particle, bead, resin, membrane, solid support or tag.

2. The polypeptide of item 1, wherein each protein domain is translationally fused to a further protein domain via said rigid amino acid linker.

3. The polypeptide of item 1 or 2, wherein said rigid amino acid linker is non-helical.

4. The polypeptide of item 3, wherein said non-helical rigid amino acid linker comprises an amino acid sequence of 6 to 21 amino acids in length.

5. The polypeptide of item 4, wherein amino acid sequence is a proline-rich amino acid sequence.

6. The polypeptide of any one of items 3 to 5, wherein said non-helical rigid amino acid linker comprises the amino acid sequence (XP)2+n, wherein X is any amino acid and n is 0 to 20.

7. The polypeptide of items 3 to 5, wherein said non-helical rigid amino acid linker comprises the amino acid sequence (XP)n, wherein X is any amino acid, except for D, E, K, or R and n is 2 to 20.

8. The polypeptide of any one of items 3 to 7, wherein said non-helical rigid amino acid linker (XP)n or (XP)2+n is flanked N-terminal and/or C-terminal by the amino acid sequence (GxSy)z.

9. The polypeptide of item 8, wherein x in the amino acid sequence (GxSy)z is 1 to 10, y in the amino acid sequence (GxSy)z is 1 to 10, z in the amino acid sequence (GxSy)z is 1 to 10.

10. The polypeptide of item 1 or 2, wherein said rigid amino acid linker is helical.

11. The polypeptide of item 10, wherein said helical amino acid linker comprises an amino acid sequence which locally forms a single a-helical domain.

12. The polypeptide of item 10 or 11, wherein said helical amino acid linker comprises an amino acid sequence of 10 to 30 amino acids in length.

13. The polypeptide of item 12, wherein said amino acid sequence comprises the amino acids E, K and/or R.

14. The polypeptide of item 12 or 13, wherein said amino acid sequence comprises (EAAAK)n (SEQ ID NO: 11).

15. The polypeptide of item 13 or 14, wherein n in the amino acid sequence (EAAAK)n (SEQ ID NO: 11) is 1 to 20.

16. The polypeptide of any one of the preceding items, wherein said protein domains are PDZ domains, PKD domains, lectins, peptides, aptamers, nano-bodies, pico-bodies, minimized binding domains, IgG binding domains, IgA binding domains or IgM binding domains.

17. The polypeptide of item 16, wherein said IgG binding domain comprises an amino acid sequence which locally forms at least one alpha-helix.

18. The polypeptide of item 16 or 17, wherein said linker is located after the amino acid sequence forming the last alpha-helix of each IgG-binding domain and before the amino acid sequence forming the first alpha-helix of said further IgG-binding domain.

19. The polypeptide of any one of items 16 to 18, wherein said IgG binding domain comprises an amino acid sequence locally forming at least two alpha-helices, preferably three alpha-helices.

20. The polypeptide of any one of items 17 to 19, wherein an alpha-helix is formed by an amino acid sequence of 10 to 14 amino acids in length.

21. The polypeptide of any one of items 17 to 20, wherein said IgG binding domain comprises an amino acid sequence further locally forming one or more beta-pleated sheets.

22. The polypeptide of any one of items 16 to 21, wherein said polypeptide comprises 2, 3, 4, 5, 6, 7, 8, 9, 10 or more IgG binding domains.

23. The polypeptide of any one of items 16 to 22, wherein said IgG binding domain is the A-domain of protein A, B-domain of protein A, C-domain of protein A, D-domain of protein A, E-domain of protein A, the Z-domain of protein A, an IgG binding domain of protein G, or an IgG binding domain of protein L.

24. The polypeptide of item 23, wherein said A-domain of protein A has an amino acid sequence which is at least 70% identical to the amino acid sequence shown in SEQ ID NO: 9, preferably the A-domain of protein A has the amino acid sequence shown in SEQ ID NO: 2.

25. The polypeptide of item 23, wherein said B-domain of protein A has an amino acid sequence which is at least 70% identical to the amino acid sequence shown in SEQ ID NO: 9, preferably the B-domain of protein A has the amino acid sequence shown in SEQ ID NO: 3.

26. The polypeptide of item 23, wherein said C-domain of protein A has an amino acid sequence which is at least 70% identical to the amino acid sequence shown in SEQ ID NO: 9, preferably the C-domain of protein A has the amino acid sequence shown in SEQ ID NO: 4.

27. The polypeptide of item 23, wherein said D-domain of protein A has an amino acid sequence which is at least 70% identical to the amino acid sequence shown in SEQ ID NO: 9, preferably the D-domain of protein A has the amino acid sequence shown in SEQ ID NO: 5.

28. The polypeptide of item 23, wherein said E-domain of protein A has an amino acid sequence which is at least 70% identical to the amino acid sequence shown in SEQ ID NO: 9, preferably the E-domain of protein A has the amino acid sequence shown in SEQ ID NO: 6.

29. The polypeptide of item 23, wherein said Z-domain of protein A has an amino acid sequence which is at least 70% identical to the amino acid sequence shown in SEQ ID NO: 9, preferably the Z-domain of protein A has the amino acid sequence shown in SEQ ID NO: 7.

30. The polypeptide of item 23, wherein said Z-domain of protein A is a minimalized Z domain having the amino acid sequence shown in SEQ ID NO: 8.

31. The polypeptide of any one of items 16 to 30, wherein a naturally-occurring amino acid sequence between the last alpha-helix of an IgG-binding domain and the first alpha-helix of a further IgG-binding domain is present or absent.

32. The polypeptide of any one of items 17 to 31, wherein at least one of said IgG-binding domains contains between two alpha helices an EF-hand loop.

33. The polypeptide of any one of items 1 to 32, wherein said target protein is glycosylated.

34. The polypeptide of any one of items 1 to 32, wherein said target protein is displayed by a virus, bacterial cell, fungal cell or mammalian cell.

35. The polypeptide of any one of items 1 to 32, wherein said target protein is an antibody, Fab fragment, or Fc-fusion protein.

36. A method for producing the polypeptide of any one of items 1 to 35, comprising coupling the polypeptide as defined in any one of items 1 to 35 to a carrier material, wherein said carrier material is a particle, bead, resin, membrane, solid support or tag.

37. The method of item 36, wherein said polypeptide is produced by expressing a nucleotide sequence encoding the polypeptide as defined in any one of items 1 to 34 in a host cell or by synthesizing said polypeptide, and coupling said polypeptide to a carrier material.

38. A kit comprising the polypeptide as defined in any one of items 1 to 35 and a carrier material, wherein said carrier material is a particle, bead, resin, membrane, solid support or tag.

39. A method for producing a purified target protein comprising eluting said target protein bound to the polypeptide of any one of items 1 to 35.

40. Use of the polypeptide of any one of items 1 to 35 for the production of a purified target protein.

Additional objects, advantages, and features of this disclosure will become apparent to those skilled in the art upon examination of the following Figures, which are not intended to be limiting. Thus, it should be understood that although the present disclosure is specifically disclosed by exemplary embodiments and optional features, modification and variation of the disclosures embodied therein herein disclosed may be resorted to by those skilled in the art and that such modifications and variations are considered to be within the scope of this disclosure

Those skilled in the art will recognize, or be able to ascertain, using no more than routine experimentation, many equivalents to the preferred embodiments of the present invention described herein. Such equivalents are intended to be encompassed by the present invention.

Unless otherwise stated, the following terms used in this document, including the description and items, have the definitions given below.

It is to be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the preferred embodiments of the present invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

Throughout this specification and the items which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the item element. When used herein, "consisting essentially of" does not exclude materials or steps that do not materially affect the basic and novel characteristics of the item.

In each instance herein, any of the terms "comprising", "consisting essentially of" and "consisting of" may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the items.

Other embodiments are within the following items. In addition, where features or aspects of the present invention are described in terms of Markush groups, those skilled in the art will recognize that the present invention is also thereby described in terms of any individual member or subgroup of members of the Markush group.

All publications cited throughout the text of this specification (including all patents, patent applications, scientific publications, manufacturer's specifications, instructions, etc.) are hereby incorporated by reference in their entirety. Nothing herein is to be construed as an admission that the present invention is not entitled to antedate such disclosure by virtue of prior invention. To the extent the material incorporated by reference contradicts or is inconsistent with this specification, the specification will supersede any such material.

## EXAMPLES

**Example 1:** Description of ligands

By way of example, the invention provides novel interdomain linkers to join together binding domains of protein A:

The aim is to increase the distance between the IgG binding domains with a rigid linker sequence, thus achieving a higher binding capacity for IgG. To achieve this, a proline-rich sequence is inserted into the existing interdomain sequence. To demonstrate the advantage of this peptide sequence, a linker of 8 polymerised B-domains was modified. This high number of IgG-binding domains was chosen because it had previously been shown to be particularly favourable for the binding of IgG (Freiherr von Roman and Berensmeier, 2014). The B domain was chosen because it also forms the basis for the modified and mutated ligands in many commercial protein A resins. This additionally introduced, proline-rich peptide sequence was flanked by a glycine-serine dipeptide to ensure a smooth transition into the flexible wild-type sequence, although this is not mandatory. The most important feature is the rigid structure of the sequence. This was proven by analysing the insertion of a flexible sequence GGGGSGGGGS (SEQ ID NO: 21) as a control. Figure 1 summarizes the ligands that were compared to highlight the advantage of the present invention.

The vector for the B8 and B4 ligand with wildtype linker was constructed as in Freiherr von Roman and Berensmeier (2014), cited herein, into pET28a. The modified proteins (B8rigid, B8flex, B8rigid_2P, B8rigid_6P, B8rigid_helix, B4rigid, PL_B8, and PL_B8rigid) were cloned into pET24a. The expression was performed in TB medium with 30 $\mu$g mL$^{-1}$ kanamycin in baffled shake flasks. The culture was incubated at 37 °C and 220 rpm until an OD600 of 1 was reached. Then, the culture was induced with 50 $\mu$M IPTG. The flasks were incubated at 17 °C and 220 rpm for approximately 16 - 24 h. The cells containing the intracellularly produced ligands were harvested by centrifugation at 3200 g and 4 °C for 30 min. The pellets containing B8, B8rigid and B8flex were resuspended in 50 mM Tris buffer at pH 7.8 (release buffer) stored at -20 °C at least over-night in 50 mL centrifugation tubes. The freezing and thawing were exploited for the protein release. The thawed cell harvest was further diluted cold release buffer, homogenized, and centrifuged at 14000 g and 4 °C for 10 min. The supernatant was recovered, and the pellet was resuspended in cold release buffer once again. After a second centrifugation step, the supernatants of both steps were pooled and protease inhibitor (cOmplete EDTA-free tablets, Roche) and DNAseI (AppliChem) was added. This pool was slowly titrated with 1 M HCl to pH 4. Solids were removed by centrifugation (3200 g, 4 °C for 10 min). 1 mM DTT was added, and the mixture was incubated at 4°C overnight. The solution was loaded onto a CEX (cation exchange chromtaogaphy) column (Nuvia S, BioRad). Two buffers were used during the ligand purification: 20 mM phosphate pH 4 (buffer A) and 20 mM phosphate, 1 M NaCl pH 4 (buffer B). After a wash step, elution was performed from 11 to 60% B (for B8) and from 5 to 60% B (for B8rig and B8flex), respectively. The product was concentrated and further polished by size exclusion chromatography (HiLoad 16/600 Superdex 200 pg, 120 mL CV, Cytiva) until > 90% purity (determined by SDS-PAGE). The pellets containing the Protein L-based ligands (PL_B8, PL_B8rigid) were resuspended in binding buffer (50 mM Tris. 0.05 mM Tween, 150 mM NaCl, pH 7.6), disrupted by ultra sonic (Hielscher UP400ST ultrasonic processor, settings: 45% amplitude, 10s on, 10s off, 5 min in total) and the protease inhibitor was added (cOmplete EDTA-free tablets, Roche). These ligands were purified via IgG-Sepharose (Cytiva) according to the manufacturer's instructions without the intermediate pH wash step.

**Example 2:** Characterization of the polymerised B-domains B8, B8flex and B8rigid

SDS-PAGE (sodium dodecyl sulfate-polyacrylamide gel electrophoresis) (Nowakowski et al., 2014) was applied to purified ligands to verify their successful expression and purification. Therefore, 12 % acrylamide / bisacrylamide was casted and proteins were resolved using 1x running buffer SDS-Tris-glycine buffer (AppliChem) with a constant voltage of 120 V for approx. 70 min until the bromophenol blue front reached the bottom of the gel. Expression of NEB P7719 (a control marker), B8, B8rigid, and B8flex was validated and confirmed by comparison with calculated molecular weights for each polypeptide. Affinity constant ($K_D$) was determined for each ligand via surface plasmon resonance (SPR), as previously described (Douzi, 2017; Sparks et al., 2019), with $K_D$ derived from the Langmuir fit of the equilibrium data. For SPR, ligands were immobilized on the CM5 sensor chip by thiol coupling. The ligand was prepared by reducing the S-S bound homodimers. 2 g L$^{-1}$ B8 in PBS was incubated with a 100 times molar excess of TCEP for 1.5 h at RT. Subsequently, the protein was rebuffered into the coupling buffer 20 mM Na acetate pH 4.5 by centrifugal concentrators. The immobilization onto the chip was performed according to the manufacturer (see "Thiol Coupling Kit", section: "ligand thiol coupling" 22-0618-10 AB 06/2013, GE Healthcare). Different concentrations of human IgG were loaded through the channels of the chip at a flowrate of 30 $\mu$L min$^{-1}$ in duplicates. The contact time was set to 180 s, the dissociation time to 400 s. The order of the IgG samples was from the lowest to the highest concentration starting with blank injections. The regeneration between each cycle was performed with 100 mM glycine pH 1.8 for 30 s. Size exclusion-high-performance liquid chromatography (SEC-HPLC) was applied to evaluate molecular weight of B8, B8rigid, and B8flex, using a flow rate of 0.20mL min$^{-1}$ and run in a solution comprising 50mM K-phosphate and 250mM KCl, with a solution pH of 6.8. All SEC-HPLC was performed using Biozen™ SEC-3 columns. The MW was determined by SEC-HPLC through comparison with a standard of globular proteins (Supelco mix 15 - 600 kDa, Sigma Aldrich) and related to the calculated MW based on the sequence. The size of a sample was determined by firstly calculating the apparent distribution coefficient. During SEC-HPLC, proteins are separated according to their hydrodynamic diameter. For globular proteins such as those of the size standard used, the MW can be derived from the SEC. A large ratio between the calculated MW and the measured MW ($MW_{SEC}$) suggests that a polypeptide is not tightly folded in conformation - in contrast to globular proteins, which might be advantageous regarding the availability of the binding sites for a binding partner.

**Example 3:** Comparison of Dynamic Binding Capacities of Epoxy-Activated Resins and Ligands for Human IgG

Each of the analysed proteins i.e. B8, B8rigid, B8flex, PL_B8, and PL_B8rigid comprised a tripeptide of lysis-cysteine-lysine at their C-terminus to enable immobilisation to the epoxy-activated chromatography material (Freiherr von Roman and Berensmeier, 2014). The immobilisation was conducted using a 33.3% resin slurry (Profinity epoxide, Bio-Rad). The dry resin was suspended in ligand solution (5 g L$^{-1}$, prepared in H$_2$O) in Eppendorf Protein LoBind 50 mL tubes. The coupling buffer 2.8 M phosphate pH 7.4 was added 1:1 to the mixture resulting in the final conditions: 1.4 M phosphate pH 7.4; 2.5 g L-1 cys-tagged ligand; 41.67 mg mL-1 resin. The reaction was performed for at least 16 h at 25 °C. The resin was washed with H$_2$O (2 x) and with PBS (3 x). During the last wash step, half of the supernatant volume was discarded and replaced by the blocking solution ethanolamine (1.33 M) pH 8. The epoxy group inactivation was performed overnight for at least 16 h. Extensive wash steps were performed in order to remove non-covalently bound protein: 20 mM phosphate pH 7.4, 500 mM NaCl (4 x); 20 mM sodium acetate pH 4, 500 mM NaCl (4 x) and finally washed 3 x with the storage buffer PBS with sodium azide (Rockland Inc.) and stored at 4 °C upon usage. Each protein was immobilised on epoxy-activated resin and dynamic binding capacity (DBC) then calculated. For each protein, two different residence times were used in assessing DBC. For the materials containing the ligands B8, B8rigid and B8flex, the feed solution was 1g L$^{-1}$ polyclonal, human IgG (Cutaquig®, Octapharma GmbH) in PBS pH 7.4. In addition, a commercial resin (SUPrA, Bio-Rad Inc.) was also analysed under these conditions. Experiments were performed in duplicates. The dynamic binding capacities were determined at a breakthrough concentration of 10% of the feed solution (DBC10%). DBC10% was determined at two different residence times. The column diameter was 0.66 cm. The column was regenerated in between with 0.1 M Na citrate pH 3.0. The first DBC was additionally quantified by offline UV$_{280}$. Therefore, 100 μL sample as well as standard samples added to a 96 well plate (UV-Star, Greiner) and measured in the microplate reader. The UV signal from the breakthrough curves was calculated back to the concentration using the so derived standard curve. Subsequently, the breakthrough curve was fitted using a 5-parameter log-function on the software OriginPro. The DBC for IgG of the resins with immobilized PL_B8 and PL_B8rigid were analysed using 0.5 g L$^{-1}$ polyclonal human IgG. IgG was eluted by 0.1 M Na citrate pH 2.3. The effect of the non-binding lambda light chain species was subtracted from the breakthrough curves. The dynamic binding capacity (DBC) influences the productivity of the chromatography step. The productivity P can be described as follows (Perez-Almodovar and Carta, 2009):

$$P = \frac{Amount\ of\ recovered\ IgG}{Total\ cycle\ time \times Column\ volume} = \frac{DBC \times Y}{\frac{DBC}{C_F} \times \frac{L}{u_{Load}} + t_{equil} + t_{wash} + t_{elu} + t_{CIP}}$$

Where Y is the yield, CF is the IgG concentration in the initial solution and L/u$_{Load}$ is the residence time, which is calculated from the bed height L and the linear flow rate u$_{Load}$. If we now compare the different DBC10% obtained with B8cys (20.3g L$^{-1}$) and B8rigid-cys (34.4g L$^{-1}$) at a residence time of 2.1 min and an IgG feed CF of 1g L$^{-1}$, the increase in productivity can be estimated. The other parameters were kept constant (see Perez-Almodovar and Carta (2009)): $t_{equil} + t_{wash} + t_{elu} + t_{CIP} = $ 31 *min.* The effect of the invented linker sequence on the IgG binding capacity of the Protein A-based B8-ligands is seen in Figure 3. Thus, the invention has a positive effect on the overall productivity. Also, the insertion of the rigid linker into Protein L (PL_B8) exhibited a positive effect in on the DBC10%, also when normalized to molar ligand density (data not shown). It is expected that the different ligands containing different rigid linker sequences are enhancing the DBC10% for their binding partner.

**Example 4:** Simulation of the hydrodynamic diameter using prediction models

The incorporation of the proline-rich linker sequence led to an overproportionately large increase in the hydrodynamic diameter shown in the examples before, which may have contributed to the enhancement in IgG binding capacity. The impact of accompanying sequences on the hydrodynamic diameter could be further explored using AI-assisted structure prediction methods. The different combinations of domains and linker sequences that were shown in Figure 1 were simulated for their structure. AlphaFold3 (AF3) (Abramson et al. 2024) was used to model the structure of the respective antibody binding ligands. Due to the rather unstructured nature of the linker region and its ability to come in multiple conformations, accurately predicting the ligand size based on an AF3 structural model is difficult. Consequently, the confidence scores (pLDDT, Predicted Local Distance Difference Test) in this region are consistently low to very low. For that reason, AFflecto (Pajkos et al. 2025) was used to predict the different conformations resulting from the flexibility of the linker region. AFflecto is a web server-based tool for generating conformational ensembles of proteins that include both structured domains and unstructured parts from AF3 structural models. For the analysis, the most confident AF3 model was loaded to AFflecto. The respective domains were defined as rigid bodies, while the unstructured region between them was designated as the linker region. AFflecto allows for highlighting structural features (canonical secondary structural elements) within linkers. The parts containing the proline residues were annotated as a polyproline II (PPII) helix within the linker region. According to Kelly et al. (2001), the PAP sequence also enables PPII helix formation. Using AFflecto, an ensemble of 100 conformations was generated. Subsequently, the hydrodynamic diameter of these structures was estimated by predicting the Stokes radius (dh) using the HullRad (Fleming und Fleming 2018) algorithm. Figure 4A shows the resulting hydrodynamic diameters (dh) derived from the structural models. For proteins with available experimental data (SEC-HPLC, Figure 2), the simulated diameters showed good agreement, supporting the validity of the modeling approach. To assess whether the increase in hydrodynamic diameter correlates with the increase in molecular weight (MW), the percentage increase was normalized to MW (Figure 4B). The results indicate that the addition of $GS(AP)_4SG$ leads to a significantly greater increase in diameter compared to $(AP)_2$, particularly in ligands containing eight B-domains. Adding two more AP repeats resulted in only a slight normalized increase. This trend was also observed in constructs with a reduced number of B-domains (4 domains) and in Protein L (8 domains), although to a lesser extent. These findings suggest that the inclusion of the sequences provides structural benefits across different domain numbers and types and may so enhance binding capacity for their binding partners.

## References

Abramson, Josh; Adler, Jonas; Dunger, Jack; Evans, Richard; Green, Tim; Pritzel, Alexander et al. 2024. Accurate structure prediction of biomolecular interactions with AlphaFold 3. Nature 630 (8016), S. 493-500. DOI: 10.1038/s41586-024-07487-w.

Amet, N., Lee, H.-F., Shen, W.-C., 2009. Insertion of the designed helical linker led to increased expression of tf-based fusion proteins. Pharm. Res. 26, 523-528. https://doi.org/10.1007/s11095-008-9767-0

Arai, R., Ueda, H., Kitayama, A., Kamiya, N., Nagamune, T., 2001. Design of the linkers which effectively separate domains of a bifunctional fusion protein. Protein Eng. 14, 529-532. https://doi.org/10.1093/protein/14.8.529

Arora, S., Saxena, V., Ayyar, B.V., 2017. Affinity chromatography: A versatile technique for antibody purification. Methods 116, 84-94. https://doi.org/10.1016/j.ymeth.2016.12.010

Bjorkman, T., Monie, E., Rodrigo, G., 2017. Affinity chromatography matrix. US9683013B2.

Cao, L., Coventry, B., Goreshnik, I., Huang, B., Sheffler, W., Park, J.S., Jude, K.M., Marković, I., Kadam, R.U., Verschueren, K.H.G., Verstraete, K., Walsh, S.T.R., Bennett, N., Phal, A., Yang, A., Kozodoy, L., DeWitt, M., Picton, L., Miller, L., Strauch, E.-M., DeBouver, N.D., Pires, A., Bera, A.K., Halabiya, S., Hammerson, B., Yang, W., Bernard, S., Stewart, L., Wilson, I.A., Ruohola-Baker, H., Schlessinger, J., Lee, S., Savvides, S.N., Garcia, K.C., Baker, D., 2022. Design of protein-binding proteins from the target structure alone. Nature 605, 551-560. https://doi.org/10.1038/s41586-022-04654-9

Chen, X., Zaro, J., Shen, W.-C., 2013. Fusion Protein Linkers: Property, Design and Functionality. Adv. Drug Deliv. Rev. 65, 1357-1369. https://doi.org/10.1016/j.addr.2012.09.039

Chettri, D., Boro, M., Sarkar, L., Verma, A.K., 2021. Lectins: Biological significance to biotechnological application. Carbohydr. Res. 506, 108367. https://doi.org/10.1016/j.carres.2021.108367

Choi, H., Park, H., Son, K., Kim, H.M., Jung, Y., 2019. Fabrication of rigidity and space variable protein oligomers with two peptide linkers. Chem. Sci. 10, 10428-10435. https://doi.org/10.1039/C9SC04158C

Dong, J., Kojima, T., Ohashi, H., Ueda, H., 2015. Optimal fusion of antibody binding domains resulted in higher affinity and wider specificity. J. Biosci. Bioeng. 120, 504-509. https://doi.org/10.1016/j.jbiosc.2015.03.014

Douzi, B., 2017. Protein-Protein Interactions: Surface Plasmon Resonance. Methods Mol. Biol. Clifton NJ 1615, 257-275. https://doi.org/10.1007/978-1-4939-7033-9_21

Fishman, J.B., Berg, E.A., 2019. Protein A and Protein G Purification of Antibodies. Cold Spring Harb. Protoc. 2019, pdb.prot099143. https://doi.org/10.1101/pdb.prot099143

Fleming, Patrick J.; Fleming, Karen G. 2018. HullRad: Fast Calculations of Folded and Disordered Protein and Nucleic Acid Hydrodynamic Properties. Biophysical journal 114 (4), S. 856-869. DOI: 10.1016/j.bpj.2018.01.002.

Freiherr von Roman, M., Berensmeier, S., 2014. Improving the binding capacities of protein A chromatographic materials by means of ligand polymerization. J. Chromatogr. A 1347, 80-86. https://doi.org/10.1016/j.chroma.2014.04.063

George, R.A., Heringa, J., 2002. An analysis of protein domain linkers: their classification and role in protein folding. Protein Eng. 15, 871-879. https://doi.org/10.1093/protein/15.11.871

Ghose, S., Allen, M., Hubbard, B., Brooks, C., Cramer, S.M., 2005. Antibody variable region interactions with Protein A: implications for the development of generic purification processes. Biotechnol. Bioeng. 92, 665-673. https://doi.org/10.1002/bit.20729

Ho, B.K., Coutsias, E.A., Seok, C., Dill, K.A., 2005. The flexibility in the proline ring couples to the protein backbone. Protein Sci. Publ. Protein Soc. 14, 1011-1018. https://doi.org/10.1110/ps.041156905

Housden, N.G., Harrison, S., Roberts, S.E., Beckingham, J.A., Graille, M., Stura, E., Gore, M.G., 2003.

Immunoglobulin-binding domains: Protein L from Peptostreptococcus magnus. Biochem. Soc. Trans. 31, 716-718. https://doi.org/10.1042/bst0310716

Huang, J., Ru, B., Zhu, P., Nie, F., Yang, J., Wang, X., Dai, P., Lin, H., Guo, F.-B., Rao, N., 2012. MimoDB 2.0: a mimotope database and beyond. Nucleic Acids Res. 40, D271-D277. https://doi.org/10.1093/nar/gkr922

Kanje, S., Scheffel, J., Nilvebrant, J., Hober, S., 2020. Engineering of Protein A for improved purification of antibodies and Fc-fused proteins, in: Approaches to the Purification, Analysis and Characterization of Antibody-Based Therapeutics. Elsevier, pp. 35-54. https://doi.org/10.1016/B978-0-08-103019-6.00002-3

Kelly, M. A.; Chellgren, B. W.; Rucker, A. L.; Troutman, J. M.; Fried, M. G.; Miller, A. F.; Creamer, T. P. 2001. Host-guest study of left-handed polyproline II helix formation. Biochemistry 40 (48), S. 14376-14383. DOI: 10.1021/bi011043a.

Lai, Y.-T., Jiang, L., Chen, W., Yeates, T.O., 2015. On the predictability of the orientation of protein domains joined by a spanning alpha-helical linker. Protein Eng. Des. Sel. PEDS 28, 491-499. https://doi.org/10.1093/protein/gzv035

Linhult, M., Gülich, S., Gräslund, T., Nygren, P., Hober, S., 2003. Evaluation of different linker regions for multimerization and coupling chemistry for immobilization of a proteinaceous affinity ligand. Protein Eng. Des. Sel. 16, 1147-1152. https://doi.org/10.1093/protein/gzg121

Ljungquist, C., Jansson, B., Moks, T., Uhlén, M., 1989. Thiol-directed immobilization of recombinant IgG-binding receptors. Eur. J. Biochem. 186, 557-561. https://doi.org/10.1111/j.1432-1033.1989.tb15244.x

Maeda, Y., Ueda, H., Kazami, J., Kawano, G., Suzuki, E., Nagamune, T., 1997. Engineering of functional chimeric protein G-Vargula luciferase. Anal. Biochem. 249, 147-152. https://doi.org/10.1006/abio.1997.2181

Matsuda, Y., 2022. Current approaches for the purification of antibody-drug conjugates. J. Sep. Sci. 45, 27-37. https://doi.org/10.1002/jssc.202100575

Nilsson, B., Moks, T., Jansson, B., Abrahmsén, L., Elmblad, A., Holmgren, E., Henrichson, C., Jones, T.A., Uhlén, M., 1987. A synthetic IgG-binding domain based on staphylococcal protein A. Protein Eng. 1, 107-113. https://doi.org/10.1093/protein/1.2.107

Nilson, B.H.K., Lögdberg, L., Kastern, W., Björck, L., Åkerström, B., 1993. Purification of antibodies using protein L-binding framework structures in the light chain variable domain. J. Immunol. Methods 164, 33-40. https://doi.org/10.1016/0022-1759(93)90273-A

Pabst, T.M., Palmgren, R., Forss, A., Vasic, J., Fonseca, M., Thompson, C., Wang, W.K., Wang, X., Hunter, A.K., 2014. Engineering of novel Staphylococcal Protein A ligands to enable milder elution pH and high dynamic binding capacity. J. Chromatogr. A 1362, 180-185. https://doi.org/10.1016/j.chroma.2014.08.046

Pabst, T.M., Thai, J., Hunter, A.K., 2018. Evaluation of recent Protein A stationary phase innovations for capture of biotherapeutics. J. Chromatogr. A 1554, 45-60. https://doi.org/10.1016/j.chroma.2018.03.060

Pajkos, M.; Clerc, I.; Zanon, C.; Bernadó, P.; Cortés, J. 2025. AFflecto: A web server to generate conformational ensembles of flexible proteins from AlphaFold models. In: Journal of Molecular Biology 437 (15), S. 169003. DOI: 10.1016/j.jmb.2025.169003.

Patel, Dharti, Menon, D., Patel, Darshan, 2020. Linkers: a synergistic way for chimeric proteins. https://doi.org/10.22541/au.160199719.94542802/v1

Perez-Almodovar, E.X., Carta, G., 2009. IgG adsorption on a new protein A adsorbent based on macroporous hydrophilic polymers. I. Adsorption equilibrium and kinetics. J. Chromatogr. A 1216, 8339-8347. https://doi.org/10.1016/j.chroma.2009.09.017

Reverdatto, S., Burz, D.S., Shekhtman, A., 2015. Peptide aptamers: development and applications. Curr. Top. Med. Chem. 15, 1082-1101. https://doi.org/10.2174/1568026615666150413153143

Ribatti, D., 2014. From the discovery of monoclonal antibodies to their therapeutic application: an historical reappraisal. Immunol. Lett. 161, 96-99. https://doi.org/10.1016/j.imlet.2014.05.010

Shen, M., Rusling, J., Dixit, C.K., 2017. Site-Selective Orientated Immobilization of Antibodies and Conjugates for Immunodiagnostics Development. Methods San Diego Calif 116, 95-111. https://doi.org/10.1016/j.ymeth.2016.11.010

Sjödahl, J., 1977. Structural studies on the four repetitive Fc-binding regions in protein A from Staphylococcus aureus. Eur. J. Biochem. 78, 471-490. https://doi.org/10.1111/j.1432-1033.1977.tb11760.x

Sparks, R.P., Jenkins, J.L., Fratti, R., 2019. Use of Surface Plasmon Resonance (SPR) to Determine Binding Affinities and Kinetic Parameters Between Components Important in Fusion Machinery. Methods Mol. Biol. Clifton NJ 1860, 199-210. https://doi.org/10.1007/978-1-4939-8760-3_12

Uhlén, M., Guss, B., Nilsson, B., Gatenbeck, S., Philipson, L., Lindberg, M., 1984. Complete sequence of the staphylococcal gene encoding protein A. A gene evolved through multiple duplications. J. Biol. Chem. 259, 1695-1702.

van Rosmalen, M., Krom, M., Merkx, M., 2017. Tuning the Flexibility of Glycine-Serine Linkers To Allow Rational Design of Multidomain Proteins. Biochemistry 56, 6565-6574. https://doi.org/10.1021/acs.biochem.7b00902

Vijayan, M., Chandra, N., 1999. Lectins. Curr. Opin. Struct. Biol. 9, 707-714. https://doi.org/10.1016/s0959-440x(99)00034-2

Walkup WG 4th, Kennedy MB. PDZ affinity chromatography: a general method for affinity purification of proteins based on PDZ domains and their ligands. Protein Expr Purif. 2014 Jun;98:46-62. doi: 10.1016/j.pep.2014.02.015.

Wilchek, M., Miron, T., 2003. Oriented versus random protein immobilization. J. Biochem. Biophys. Methods 55, 67-70. https://doi.org/10.1016/s0165-022x(02)00178-1

**Claims**

1.  A polypeptide comprising protein domains binding to a specific site on the surface of a target protein, wherein each protein domain is translationally fused in its N- to C-terminal orientation to a further protein domain that is also in its N- to C-terminal orientation, via a rigid amino acid linker keeping a fixed distance between said protein domains and said rigid amino acid linker is also in its N- to C-terminal orientation, wherein said polypeptide is coupled to a carrier material, wherein said carrier material is a particle, bead, resin, membrane, solid support or tag.

2.  The polypeptide of claim 1, wherein said rigid amino acid linker is non-helical.

3.  The polypeptide of claim 2, wherein said non-helical rigid amino acid linker comprises an amino acid sequence of 6 to 21 amino acids in length.

4.  The polypeptide of claim 2, wherein said non-helical rigid amino acid linker comprises the amino acid sequence (XP)2+n, wherein X is any amino acid and n is 0 to 20.

5.  The polypeptide of claim 2, wherein said non-helical rigid amino acid linker comprises the amino acid sequence (XP)n, wherein X is any amino acid, except for D, E, K, or R, and n is 2 to 20.

6.  The polypeptide of any one of claims 2, 4, or 5, wherein said non-helical rigid amino acid linker (XP)n or (XP)2+n is flanked N-terminal and/or C-terminal by the amino acid sequence (GxSy)z, wherein x is 1 to 10, y is 1 to 10, and z is 1 to 10.

7.  The polypeptide of claim 1, wherein said rigid amino acid linker is helical.

8.  The polypeptide of claim 7, wherein said helical amino acid linker comprises an amino acid sequence which locally forms a single a-helical domain.

9.  The polypeptide of claim 7 or 8, wherein said helical amino acid linker comprises an amino acid sequence of 10 to 30 amino acids in length.

10. The polypeptide of claim 7 or 8, wherein said helical amino acid linker comprises the amino acid sequence (EAAAK)n, wherein n is 1 to 20.

11. The polypeptide of any one of the preceding claims, wherein said protein domains are PDZ domains, PKD domains, lectins, peptides, aptamers, nano-bodies, pico-bodies, minimized binding domains, IgG binding domains, IgA binding domains or IgM binding domains.

12. The polypeptide of claim 11, wherein said polypeptide comprises 2, 3, 4, 5, 6, 7, 8, 9, 10 or more IgG binding domains.

13. The polypeptide of claim 11 or 12, wherein said IgG binding domain is the A-domain of protein A, B-domain of protein A, C-domain of protein A, D-domain of protein A, E-domain of protein A, the Z-domain of protein A, an IgG binding domain of protein G, or an IgG binding domain of protein L.

14. The polypeptide of any one of claims 1 to 13, wherein said target protein is an antibody, Fab fragment, or Fc-fusion protein.

15. A method for producing a purified target protein comprising eluting said target protein bound to the polypeptide of any one of claims 1 to 14.

## Figure 1

| Ligand | Description | Sequence between B-domains / Linker sequences in bold | MW$_{calc.}$, kDa |
|---|---|---|---|
| B8rigid | 8 B-domains from Protein A with rigid interdomain sequence (invention) | QAPK**GSAPAPAPAPASG**ARADNKFHK (SEQ ID NO: 13)<br><br>Linker:<br>**GSAPAPAPAPASG** (SEQ ID NO: 20) | 62.9 |
| B8 | 8 B-domains from Protein A polymerised * (control) | QAPKILE------------ADNKFHK (SEQ ID NO: 14)<br><br>Linker:<br>No linker used | 55.8 |
| B8flex | 8 B-domains from Protein A with flexible interdomain sequence (control) | QAPK**GGGGSGGGGS**---ARADNKFHK (SEQ ID NO: 15)<br><br>Linker:<br>**GGGGSGGGGS** (SEQ ID NO: 21) | 59.6 |
| B8rigid_2P | 8 B-domains from Protein A with rigid interdomain sequence (invention) | QAPK**GSAPAPASG**ARADNKFHK (SEQ ID NO: 23)<br><br>Linker:<br>**GSAPAPASG** (SEQ ID NO: 24) | 59.0 |
| B8rigid_6P | 8 B-domains from Protein A with rigid interdomain sequence (invention) | QAPK**GSAPAPAPAPAPAPASG**ARADNKFHK (SEQ ID NO: 25)<br><br>Linker:<br>**GSAPAPAPAPAPAPASG** (SEQ ID NO: 26) | 64.4 |
| B8rigid_helix | 8 B-domains from Protein A with rigid interdomain sequence (invention) | QAPK**GSEAAAKEAAAKEAAAKSG**ARADNK (SEQ ID NO: 27)<br><br>Linker:<br>**GSEAAAKEAAAKEAAAKSG** (SEQ ID NO: 28) | 67.1 |
| B4 | 4 B-domains from Protein A (control) | QAPKILE------------ADNKFHK (SEQ ID NO: 14)<br><br>Linker:<br>No linker used | 28.1 |
| B4rigid | 4 B-domains from Protein A with rigid interdomain sequence (invention) | QAPK**GSAPAPAPAPASG**ARADNKFHK (SEQ ID NO: 13)<br><br>Linker:<br>**GSAPAPAPAPASG** (SEQ ID NO: 20) | 31.8 |
| PL_B8 | 8 B3-domains* from Protein A (control) (SEQ ID NO: 29) | YTLNIKFA------------GKEKTPEEP (SEQ ID NO: 30)<br><br>Linker: | 64.7 |

| | | No linker used | |
|---|---|---|---|
| **PL_B8rigid** | 8 B3-domains* from Protein L with rigid interdomain sequence (invention) | YTLNIKFA**GSAPAPAPAPASG** GKEKTPEEP (SEQ ID NO: 31)<br><br>Linker:<br>**GSAPAPAPAPASG** (SEQ ID NO: 20) | 72.4 |

*Including point mutation for stability

**Figure 2**

**Figure 3A**

| Protein | Ligand density, mg mL$^{-1}$ | Colum I.D., cm | Bed height, cm | Column volume, µL |
|---|---|---|---|---|
| B8 | 1.84±0.02 | 0.66 | 1.23 | 419 |
| B8rigid | 1.97±0.01 | 0.66 | 1.23 | 419 |
| B8flex | 2.92±0.01 | 0.66 | 1.23 | 419 |
| SUPrA (Bio-Rad) | 4.64±0.24 | 0.66 | 1.35 | 462 |
| PL-B8 | 3.40±0.03 | 0.66 | 1.12 | 382 |
| PL-B8rigid | 3.99±0.11 | 0.66 | 1.16 | 399 |

**Figure 3B**

**Figure 3C**

**Figure 4A**

**Figure 4B**

percentual increase in diameter normalized to MW

Europäisches Patentamt
European Patent Office
Office européen des brevets

# EUROPEAN SEARCH REPORT

**Application Number**

EP 25 20 9828

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FREIHERR VON ROMAN MATTHIAS ET AL: "Improving the binding capacities of protein A chromatographic materials by means of ligand polymerization", JOURNAL OF CHROMATOGRAPHY A, ELSEVIER, AMSTERDAM, NL, vol. 1347, 28 April 2014 (2014-04-28), pages 80-86, XP029026847, ISSN: 0021-9673, DOI: 10.1016/J.CHROMA.2014.04.063 * from the inventor; page 81, column 1 * | 1-15 | INV. C07K16/00 C07K17/00 |
| Y | XIAOYING CHEN ET AL: "Fusion protein linkers: Property, design and functionality", ADVANCED DRUG DELIVERY REVIEWS, 1 September 2012 (2012-09-01), XP055062583, ISSN: 0169-409X, DOI: 10.1016/j.addr.2012.09.039 * page 5; tables 2,3 * | 1-15 | |

-----

-----

-/--

**TECHNICAL FIELDS SEARCHED (IPC)**

C07K

The present search report has been drawn up for all claims

| Place of search | Date of completion of the search | Examiner |
|---|---|---|
| Munich | 12 February 2026 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European
Patentamt

European
Patent Office

Office européen
des brevets

# EUROPEAN SEARCH REPORT

**Application Number**

EP 25 20 9828

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | "Approaches to the Purification, Analysis and Characterization of Antibody-Based Therapeutics", Elsevier , 1 January 2020 (2020-01-01), pages 35-54, XP093089837, DOI: 10.1016/B978-0-08-103019-6.00002-3 ISBN: 978-0-08-103019-6 Retrieved from the Internet: URL:https://pdf.sciencedirectassets.com/77 8504/3-s2.0-C20180027437/3-s2.0-B978008103 0196000023/main.pdf?X-Amz-Security-Token=I QoJb3JpZ2luX2VjELT//////////wEaCXVzLWVhc3Q tMSJHMEUCIQCeaErqi3CS9emOY2BZQ+11AVI0Qm5dl 0GJvh2Hs+zjdwIgWKa00Iz7LX8DuYKoq+wI9LhKKSa vJJeAxP03I4YT/jIqvAUIvP//////////ARAFGgwwN TkwMDM1NDY * the whole document * | 1-15 | |
| A | RYOICHI ARAI: "Design of Helical Linkers for Fusion Proteins and Protein-Based Nanostructures", 21 January 2021 (2021-01-21), LINKERS IN BIOMACROMOLECULES; METHODS IN ENZYMOLOGY,, PAGE(S) 209 - 230, XP009548593, ISBN: 978-0-12-820818-2 [retrieved on 2020-11-18] * the whole document * | 1-15 | TECHNICAL FIELDS SEARCHED (IPC) |
| A | WO 2009/142773 A2 (BRISTOL MYERS SQUIBB CO [US]; CAMPHAUSEN RAY [US] ET AL.) 26 November 2009 (2009-11-26) * page 71 - page 72; sequences 60-62,65-71 * | 1-15 | |

The present search report has been drawn up for all claims

| Place of search | Date of completion of the search | Examiner |
|---|---|---|
| Munich | 12 February 2026 | Luo, Xinmei |

EPO FORM 1503 03.82 (P04C01)

page 2 of 2

## ANNEX TO THE EUROPEAN SEARCH REPORT
## ON EUROPEAN PATENT APPLICATION NO.

EP 25 20 9828

This annex lists the patent family members relating to the patent documents cited in the above-mentioned European search report.
The members are as contained in the European Patent Office EDP file on
The European Patent Office is in no way liable for these particulars which are merely given for the purpose of information.

12-02-2026

| Patent document cited in search report | Publication date | Patent family member(s) | | Publication date |
|---|---|---|---|---|
| WO 2009142773 A2 | 26-11-2009 | AR | 071874 A1 | 21-07-2010 |
| | | CL | 2009001258 A1 | 10-09-2010 |
| | | CN | 102099373 A | 15-06-2011 |
| | | EP | 2291399 A2 | 09-03-2011 |
| | | EP | 2799448 A1 | 05-11-2014 |
| | | JP | 2011520961 A | 21-07-2011 |
| | | PE | 20091931 A1 | 31-12-2009 |
| | | TW | 201000118 A | 01-01-2010 |
| | | US | 2009299040 A1 | 03-12-2009 |
| | | US | 2013012435 A1 | 10-01-2013 |
| | | US | 2014349929 A1 | 27-11-2014 |
| | | US | 2018265572 A1 | 20-09-2018 |
| | | US | 2021017252 A1 | 21-01-2021 |
| | | WO | 2009142773 A2 | 26-11-2009 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 24207783
- WO 2018046475 A
- US 5525711 A
- US 4711955 A
- US 5792608 A
- EP 302175 A
- US 6399750 B
- WO 2011073389 A
- US 9683013 B2

**Non-patent literature cited in the description**

- **ABRAMSON, JOSH** ; **ADLER, JONAS** ; **DUNGER, JACK** ; **EVANS, RICHARD** ; **GREEN, TIM** ; **PRITZEL, ALEXANDER et al.** Accurate structure prediction of biomolecular interactions with AlphaFold 3.. *Nature*, 2024, vol. 630 (8016), 493-500
- **AMET, N.** ; **LEE, H.-F.** ; **SHEN, W.-C.** Insertion of the designed helical linker led to increased expression of tf-based fusion proteins.. *Pharm. Res.*, 2009, vol. 26, 523-528
- **ARAI, R.** ; **UEDA, H.** ; **KITAYAMA, A.** ; **KAMIYA, N.** ; **NAGAMUNE, T.** Design of the linkers which effectively separate domains of a bifunctional fusion protein.. *Protein Eng.*, 2001, vol. 14, 529-532
- **ARORA, S.** ; **SAXENA, V.** ; **AYYAR, B.V.** Affinity chromatography: A versatile technique for antibody purification.. *Methods*, 2017, vol. 116, 84-94
- **BJORKMAN, T.** ; **MONIE, E.** ; **RODRIGO, G.** *Affinity chromatography matrix.*, 2017
- **CAO, L.** ; **COVENTRY, B.** ; **GORESHNIK, I.** ; **HUANG, B.** ; **SHEFFLER, W.** ; **PARK, J.S.** ; **JUDE, K.M.** ; **MARKOVIĆ, I.** ; **KADAM, R.U.** ; **VERSCHUEREN, K.H.G.** Design of protein-binding proteins from the target structure alone.. *Nature*, 2022, vol. 605, 551-560
- **CHEN, X.** ; **ZARO, J.** ; **SHEN, W.-C.** Fusion Protein Linkers: Property, Design and Functionality.. *Adv. Drug Deliv. Rev.*, 2013, vol. 65, 1357-1369
- **CHETTRI, D.** ; **BORO, M.** ; **SARKAR, L.** ; **VERMA, A.K.** Lectins: Biological significance to biotechnological application.. *Carbohydr. Res.*, 2021, vol. 506, 108367
- **CHOI, H.** ; **PARK, H.** ; **SON, K.** ; **KIM, H.M.** ; **JUNG, Y.** Fabrication of rigidity and space variable protein oligomers with two peptide linkers.. *Chem. Sci.*, 2019, vol. 10, 10428-10435
- **DONG, J.** ; **KOJIMA, T.** ; **OHASHI, H.** ; **UEDA, H.** Optimal fusion of antibody binding domains resulted in higher affinity and wider specificity.. *J. Biosci. Bioeng.*, 2015, vol. 120, 504-509
- **DOUZI, B.** Protein-Protein Interactions: Surface Plasmon Resonance.. *Methods Mol. Biol. Clifton NJ*, 2017, vol. 1615, 257-275
- **FISHMAN, J.B.** ; **BERG, E.A.** Protein A and Protein G Purification of Antibodies.. *Cold Spring Harb. Protoc.*, 2019
- **FLEMING, PATRICK J.** ; **FLEMING, KAREN G.** HullRad: Fast Calculations of Folded and Disordered Protein and Nucleic Acid Hydrodynamic Properties.. *Biophysical journal*, 2018, vol. 114 (4), 856-869
- **FREIHERR VON ROMAN, M.** ; **BERENSMEIER, S.** Improving the binding capacities of protein A chromatographic materials by means of ligand polymerization.. *J. Chromatogr. A*, 2014, vol. 1347, 80-86
- **GEORGE, R.A.** ; **HERINGA, J.** An analysis of protein domain linkers: their classification and role in protein folding.. *Protein Eng.*, 2002, vol. 15, 871-879
- **GHOSE, S.** ; **ALLEN, M.** ; **HUBBARD, B.** ; **BROOKS, C.** ; **CRAMER, S.M.** Antibody variable region interactions with Protein A: implications for the development of generic purification processes.. *Biotechnol. Bioeng.*, 2005, vol. 92, 665-673
- **HO, B.K.** ; **COUTSIAS, E.A.** ; **SEOK, C.** ; **DILL, K.A.** The flexibility in the proline ring couples to the protein backbone.. *Protein Sci. Publ. Protein Soc.*, 2005, vol. 14, 1011-1018
- **HOUSDEN, N.G.** ; **HARRISON, S.** ; **ROBERTS, S.E.** ; **BECKINGHAM, J.A.** ; **GRAILLE, M.** ; **STURA, E.** ; **GORE, M.G.** Immunoglobulin-binding domains: Protein L from Peptostreptococcus magnus.. *Biochem. Soc. Trans.*, 2003, vol. 31, 716-718
- **HUANG, J.** ; **RU, B.** ; **ZHU, P.** ; **NIE, F.** ; **YANG, J.** ; **WANG, X.** ; **DAI, P.** ; **LIN, H.** ; **GUO, F.-B.** ; **RAO, N.** MimoDB 2.0: a mimotope database and beyond.. *Nucleic Acids Res.*, 2012, vol. 40, D271-D277

- **KANJE, S.** ; **SCHEFFEL, J.** ; **NILVEBRANT, J.** ; **HOBER, S.** Engineering of Protein A for improved purification of antibodies and Fc-fused proteins, in: Approaches to the Purification, Analysis and Characterization of Antibody-Based Therapeutics.. Elsevier, 2020, 35-54
- **KELLY, M. A.** ; **CHELLGREN, B. W.** ; **RUCKER, A. L.** ; **TROUTMAN, J. M.** ; **FRIED, M. G.** ; **MILLER, A. F.** ; **CREAMER, T. P.** Host-guest study of left-handed polyproline II helix formation.. *Biochemistry*, 2001, vol. 40 (48), 14376-14383
- **LAI, Y.-T.** ; **JIANG, L.** ; **CHEN, W.** ; **YEATES, T.O.** On the predictability of the orientation of protein domains joined by a spanning alpha-helical linker.. *Protein Eng. Des. Sel. PEDS*, 2015, vol. 28, 491-499
- **LINHULT, M.** ; **GÜLICH, S.** ; **GRÄSLUND, T.** ; **NYGREN, P.** ; **HOBER, S.** Evaluation of different linker regions for multimerization and coupling chemistry for immobilization of a proteinaceous affinity ligand.. *Protein Eng. Des. Sel.*, 2003, vol. 16, 1147-1152
- **LJUNGQUIST, C.** ; **JANSSON, B.** ; **MOKS, T.** ; **UHLÉN, M.** Thiol-directed immobilization of recombinant IgG-binding receptors.. *Eur. J. Biochem.*, 1989, vol. 186, 557-561
- **MAEDA, Y.** ; **UEDA, H.** ; **KAZAMI, J.** ; **KAWANO, G.** ; **SUZUKI, E.** ; **NAGAMUNE, T.** Engineering of functional chimeric protein G-Vargula luciferase.. *Anal. Biochem.*, 1997, vol. 249, 147-152
- **MATSUDA, Y.** Current approaches for the purification of antibody-drug conjugates.. *J. Sep. Sci.*, 2022, vol. 45, 27-37
- **NILSSON, B.** ; **MOKS, T.** ; **JANSSON, B.** ; **ABRAHMSÉN, L.** ; **ELMBLAD, A.** ; **HOLMGREN, E.** ; **HENRICHSON, C.** ; **JONES, T.A.** ; **UHLÉN, M.** A synthetic IgG-binding domain based on staphylococcal protein A.. *Protein Eng.*, 1987, vol. 1, 107-113
- **NILSON, B.H.K.** ; **LÖGDBERG, L.** ; **KASTERN, W.** ; **BJÖRCK, L.** ; **ÅKERSTRÖM, B.** Purification of antibodies using protein L-binding framework structures in the light chain variable domain.. *J. Immunol. Methods*, 1993, vol. 164, 33-40
- **PABST, T.M.** ; **PALMGREN, R.** ; **FORSS, A.** ; **VASIC, J.** ; **FONSECA, M.** ; **THOMPSON, C.** ; **WANG, W.K.** ; **WANG, X.** ; **HUNTER, A.K.** Engineering of novel Staphylococcal Protein A ligands to enable milder elution pH and high dynamic binding capacity.. *J. Chromatogr. A*, 2014, vol. 1362, 180-185
- **PABST, T.M.** ; **THAI, J.** ; **HUNTER, A.K.** Evaluation of recent Protein A stationary phase innovations for capture of biotherapeutics.. *J. Chromatogr. A*, 2018, vol. 1554, 45-60
- **PAJKOS, M.** ; **CLERC, I.** ; **ZANON, C.** ; **BERNADÓ, P.** ; **CORTÉS, J.** AFflecto: A web server to generate conformational ensembles of flexible proteins from AlphaFold models.. *Journal of Molecular Biology*, 2025, vol. 437 (15), 169003
- **PATEL, DHARTI** ; **MENON, D.** ; **PATEL, DARSHAN**. *Linkers: a synergistic way for chimeric proteins.*, 2020
- **PEREZ-ALMODOVAR, E.X.** ; **CARTA, G.** IgG adsorption on a new protein A adsorbent based on macroporous hydrophilic polymers. I. Adsorption equilibrium and kinetics.. *J. Chromatogr. A*, 2009, vol. 1216, 8339-8347
- **REVERDATTO, S.** ; **BURZ, D.S.** ; **SHEKHTMAN, A.** Peptide aptamers: development and applications.. *Curr. Top. Med. Chem.*, 2015, vol. 15, 1082-1101
- **RIBATTI, D.** From the discovery of monoclonal antibodies to their therapeutic application: an historical reappraisal.. *Immunol. Lett.*, 2014, vol. 161, 96-99
- **SHEN, M.** ; **RUSLING, J.** ; **DIXIT, C.K.** Site-Selective Orientated Immobilization of Antibodies and Conjugates for Immunodiagnostics Development.. *Methods San Diego Calif*, 2017, vol. 116, 95-111
- **SJÖDAHL, J.** Structural studies on the four repetitive Fc-binding regions in protein A from Staphylococcus aureus.. *Eur. J. Biochem.*, 1977, vol. 78, 471-490
- **SPARKS, R.P.** ; **JENKINS, J.L.** ; **FRATTI, R.** Use of Surface Plasmon Resonance (SPR) to Determine Binding Affinities and Kinetic Parameters Between Components Important in Fusion Machinery.. *Methods Mol. Biol.*, 2019, vol. 1860, 199-210
- **UHLÉN, M.** ; **GUSS, B.** ; **NILSSON, B.** ; **GATENBECK, S.** ; **PHILIPSON, L.** ; **LINDBERG, M.** Complete sequence of the staphylococcal gene encoding protein A.. *A gene evolved through multiple duplications. J. Biol. Chem.*, 1984, vol. 259, 1695-1702
- **VAN ROSMALEN, M.** ; **KROM, M.** ; **MERKX, M.** Tuning the Flexibility of Glycine-Serine Linkers To Allow Rational Design of Multidomain Proteins.. *Biochemistry*, 2017, vol. 56, 6565-6574
- **VIJAYAN, M.** ; **CHANDRA, N.** *Lectins. Curr. Opin. Struct. Biol.*, 1999, vol. 9, 707-714
- **WALKUP WG 4TH** ; **KENNEDY MB.** PDZ affinity chromatography: a general method for affinity purification of proteins based on PDZ domains and their ligands.. *Protein Expr Purif.*, June 2014, vol. 98, 46-62
- **WILCHEK, M.** ; **MIRON, T.** Oriented versus random protein immobilization.. *J. Biochem. Biophys. Methods*, 2003, vol. 55, 67-70